# EUROPEAN PATENT APPLICATION

(11) **EP 1 748 070 A1**
(43) Date of publication of application: **31.01.2007**
(21) Application number: 05736570.2
(22) Date of filing: 25.04.2005
(51) Int. Cl.: C12N 15/09, A61K 38/00, A61K 39/395, A61P 19/02, A61P 29/00, A61P 35/02, A61P 37/02, C07K 16/28, C07K 19/00, C12N 15/02, C12P 21/08

(54) **THERAPEUTIC MEDICINE CONTAINING MONOCLONAL ANTIBODY AGAINST FOLATE RECEPTOR BETA (FR-BETA)**

(30) Priority: 26.04.2004 JP 2004129134
(71) Applicant: Matsuyama Takami, Kagoshima-shi, Kagoshima 8908544 (JP)
(72) Inventor: MATSUYAMA, Takami, c/o KAGOSHIMA UNIVERSITY, Kagoshima-shi, Kagoshima 8908544 (JP); NAGAYOSHI, Ryusaku, c/o KAGOSHIMA UNIVERSITY, Kagoshima-shi, Kagoshima 8908544 (JP); TSUNEYOSHI, Yasuhiro, c/o KAGOSHIMA UNIVERSITY, Kagoshima-shi, Kagoshima 8908544 (JP); NAGAI, Taku, c/o KAGOSHIMA UNIVERSITY, Kagoshima-shi, Kagoshima 8908544 (JP); MATSUSHITA, Kakushi, c/o KAGOSHIMA UNIVERSITY, Kagoshima-shi, Kagoshima 8908544 (JP)
(74) Representative: Gillard, Richard Edward
(86) International application number: PCT/JP2005/008372
(87) International publication number: WO 2005/103250

(57) **Abstract**

An objective of the present invention is to provide a therapeutic agent for treating rheumatoid arthritis, juvenile rheumatoid arthritis, macrophage activation syndrome, septicemia, and FR-β expressing leukemia, which induces apoptosis in activated macrophages and folate receptor beta (FR-β) expressing leukemia cells to specifically destroy these cells. An FR-β monoclonal antibody of the present invention is preferably an IgG-type monoclonal antibody which specifically reacts with a human-type FR-β antigen and is produced from a clone resulting from immunization with an FR-β expressing B300-19 cell. The FR-β monoclonal antibody of the present invention specifically reacts with the FR-β antigen of activated macrophages and FR-β expressing leukemia cells and a therapeutic agent of the present invention contains an FR-β antibody immunotoxin which causes apoptosis in activated macrophages and FR-β expressing leukemia cells, as an active ingredient. Further, suitable administration form for the therapeutic agent of the present invention includes Intravenous injection as well as joint injection in the case of therapeutic agents for rheumatoid arthritis and juvenile arthritis.

## Description

### Field of the Invention

The present invention relates to a composition and a method using an immunotoxin specific to a cell expressing folate receptor beta (FR-β) for treating a disease, in which the major pathological condition is macrophage activation, or leukemia expressing FR-β. More specifically, the present invention relates to development of therapy with an immunotoxin in which a toxin is bound to a monoclonal antibody against an FR-β antigen in treating rheumatoid arthritis, juvenile rheumatoid arthritis, macrophage activation syndrome, septic shock, and acute myeloid leukemia.

### Background Art

### Monoclonal antibodies

Monoclonal antibodies are produced using the method of Kohler and Milstein or a modified method thereof (Kohler et al. Continuous cultures of fused cells secreting antibody of predefined specificity. Nature. 1975 Aug 7, 256(5517):495-7) (Non-patent Reference 1).

### Toxins

A bacterial toxin, Pseudomonas exotoxin (PE), becomes active when its amino acid sequence is cleaved between 279 and 280 (Ogata et al. Cell-mediated cleavage of Pseudomonas exotoxin between Arg279 and Gly280 generates the enzymatically active fragment which translocates to the cytosol. J Biol Chem. 1992, 267(35):25396-401 (Non-patent Reference1).

A genetically engineered PE lacks the cell-surface binding Ia domain and consists of amino acids starting from position 280 of the amino acid sequence. Further, KDEL and REDLK are added to the C-terminal site to increase the cytotoxicity (Kreitman. Chimeric fusion proteins-Pseudomonas exotoxin-based. Curr Opin Investig Drugs. 2001, 2(9):1282-93)

### (Non-patent Reference 3).

Further, there have been reports on preclinical studies using various different immunotoxins which including type-1 ribosome other than PE (momordin, gelonin, saporin, bryodin, and bouganin) (Pastan I. Immunotoxins containing Pseudomonas exotoxin A: a short history. Cancer Immunol Immunother. 2003, 52(5):338-41 (Non-patent Reference 4); Trail et al. Monoclonal antibody drug immunoconjugates for targeted treatment of cancer. Cancer Immunol Immunother. 2003 May, 52(5):328-37 (Non-patent Reference 5); Milenic DE. Monoclonal antibody-based therapy strategies: providing options for the cancer patient. Curr Pharm Des. 2002, 8(19):1749-64 (Non-patent Reference 6)).

### Immunotoxins

To date, a number of immunotoxins which use recombinant PE have been disclosed.

U.S. Patent No. 6,703,488 (Patent Reference 1) describes the construction of a conjugate of an anti-IL-13 receptor antibody with a toxin in claim 6 and construction of a recombinant toxin of an anti-IL-13 receptor antibody with a genetically engineered PE40 in Example 1.

U.S. Patent No. 6,703,020 (Patent Reference 2) describes the construction of a conjugate of an anti-VEGF receptor antibody with PE in claim 16.

U.S. Patent No. 6,696,064 (Patent Reference 3) describes the construction of a conjugate of an anti-transferrin receptor antibody with a genetically engineered PE 38 in claim 6.

U.S. Patent No. 6,689,869 (Patent Reference 4) describes the construction of a conjugate of an anti-CD18 antibody with an enzyme inhibitor in claim 2 and the construction of a conjugate of an anti-CD18 antibody with PE in the specification,

U.S. Patent No. 6,417,337 (Patent Reference 5) describes the construction of a conjugate of an anti-CEA antibody with a toxin in claim 5 and its specification describes that the toxin includes PE.

U.S. Patent No. 6,395,276 (Patent Reference 6) describes the construction of a conjugate of an anti-CD22 antibody with a toxin in claim 1 and a survival prolongation effect of an anti-CD22 antibody-genetically engineered PE conjugate in Daudi cell-implanted SCID mice in Example 5.

U.S. Patent No. 6,348,581 (Patent Reference 7) describes the construction of a conjugate of an anti-TAG-72 antibody with a toxin in claim 4 and its text describes that the toxin includes PE.

U.S. Patent No. 6,346,248 (Patent Reference 8) describes the construction of a conjugate of an anti-CD86 antibody with a toxin in claim 1 and its text describes that the toxin includes PE.

U.S. Patent No. 6,319,891 (Patent Reference 9) describes the construction of a conjugate of an anti-glutathion-S-transferase antibody with PE in claim 7.

U.S. Patent No. 6,312,694 (Patent Reference 10) describes the construction of a conjugate of an anti-aminophospholipid antibody with PE in claim 31.

U.S. Patent No. 6,287,562 (Patent Reference 11) describes the construction of a conjugate of an anti-Lewis Y antibody with PE in claim 4 and suppression of cell line growth by a conjugate of an anti-Lewis Y antibody with a genetically engineered PE38 or its recombinant single chain immunotoxin in Example 7.

U.S. Patent No. 6,267,960 (Patent Reference 12) describes the construction of a conjugate of an anti-prostate stem cell antigen antibody with PE or a genetically engineered PE40 in claim 4.

U.S. Patent No. 6,074,644 (Patent Reference 13) describes in claim 1 the construction of a recombinant double chain immunotoxin by S-S bonds between a genetically engineered PE (which lacks amino acid residues 1 through 279 and half or more of domain Ib) and a protein comprising an antibody component VH or VL and a protein comprising an antibody component VL or VH, and its claim 3 describes that these antibody components comprise PE and antibody components to B1, B3, B5, e23, BR96, Tac, RFB4, and HB21.

U.S. Patent No. 6,033,876 (Patent Reference 14) describes the construction of a conjugate of an anti-CD30 antibody with a toxin in claim 4 and its text describes that the toxin includes PE38 and PE40.

U.S. Patent No. 5,980,895 (Patent Reference 15) describes in claim 1 the construction of a recombinant double chain immunotoxin in which a conjugate of an antibody component VH with a genetically engineered PE (which lacks amino acid residues 1 through 279 and half or more of domain Ib) is linked with a conjugate of an antibody component VL by S-S bonds and its claim 3 describes that the VH and VL of this recombinant immunotoxin are derived from B1, B3, B5, e23, BR96, Tac, RFB4, and HB21 antibodies.

U.S. Patent No. 5,840,854 (Patent Reference 16) describes the construction of a conjugate of an anti-GA733-1 antibody with a toxin in claim 21 and its text describes that the toxin includes PE.

U.S. Patent No. 5,817,313 (Patent Reference 17) describes the construction of a conjugate of an anti-K1(CA125) antibody with a toxin in claim 3 and the binding activity of an anti-K1(CA125) antibody-PE to OVCAR-3 cells in Table 7.

U.S. Patent No. 5,776,427 (Patent Reference 18) describes in claim 18 the construction of a conjugate of PE with each of CD5, CD8, CD11/CD18, CD15, CD32, CD44, CD45, CD64, CD25, CD30, CD54, CD71, HMFG-2, SM-3, B72.3, PR5c5, RR402, 27, OV-TL3, Mov18, and P185(HER2) antibodies.

U.S. Patent No. 5,759,546 (Patent Reference 19) describes the construction of a conjugate of an anti-CD4 antibody with a toxin in claim 11 and its text describes that the toxin includes PE.

U.S. Patent No. 5,506,343 (Patent Reference 20) describes the construction of a conjugate of an anti-unglycosylated DF3 antibody with a toxin in claim 12 and its text describes that the toxin includes PE.

U.S. Patent No. 5,045,451 (Patent Reference 21) describes the construction of a conjugate of a toxin with each of CD2, CD3, CD5, CD7, CD8, glycophorin, Thy1.1, and CD22 antibodies in claim 1 and its text describes that the toxin includes PE.

U.S. Patent No. 4,806,494 (Patent Reference 22) describes the construction of a conjugate of an anti-ovarian cancer (OVB-3) antibody with PE in claim 2.

U.S. Patent No. 4,545,985 (Patent Reference 23) describes the construction of a conjugate of each of anti-TAC and anti-transferrin receptor antibodies with PE in claim 3.

European Patent relating WO 99/64073 (Patent Reference 24) describes the construction of a conjugate of an anti-HIVGP120 antibody with a genetically engineered PE in claim 2.

European Patent relating No. NZ336576 (Patent Reference 25) describes the construction of a conjugate of each of EGP2, MUC1, MUC2, and MUC3 antibodies with PE in its abstract.

European Patent relating No. CN1330081 (Patent Reference 26) describes the construction of a conjugate of an anti-HIV antibody with a recombinant PE in its abstract.

European Patent relating WO 97/13529 (Patent Reference 27) describes in claim 1 the preparation of a recombinant double chain immunotoxin in which a protein constructed by a PE (which lacks amino acid residues 1 through 279) gene and an antibody VH gene, and an antibody VL protein are linked via S-S bonds and its claim describes that these antibodies are B1, B3, B5, e23, BR96, Tac, and HB21.

European Patent relating WO 98/41641 (Patent Reference 28) describes a method of the construction of a recombinant double chain immunotoxin in which a protein constructed by an anti-CD22 antibody VH and PE38 gene and an anti-CD22 antibody VL protein are linked via S-S bonds in claim 37 and an antitumor effect of this recombinant double chain immunotoxin in mice implanted with CD22 expressing human B-cell tumor in Example 8.

European Patent relating WO 94/13316 (Patent Reference 29) describes in claim 21 the construction of a conjugate of an antibody with a genetically engineered PE in which a mutation is introduced into domain 1 to weaken the binding to a cell and cysteine residues are added to domains 2 and 3 for binding to the antibody.

European Patent, EP 0583794 (Patent Reference 30), describes in claim 6 the construction of a conjugate of an antibody with a genetically engineered PE in which a mutation is introduced into a cell binding site of domain 1A and most or a part of domain II is deleted.

### Genetically engineered antibodies

Further, a recombinant single chain immunotoxin can be constructed by linking DNA of an antigen binding site of the H chain or L chain of an antibody and a toxin DNA by genetic manipulation and producing a protein in cells of E. coli (Haasan R et al. Antitumor activity of SS(dsFv)PE38 and SS1(dsFv)PE38, recombinant antimesothelin immunotoxins against human gynecologic cancers grown in organotypic culture in vitro. Clin Cancer Res. 2002 Nov; 8(11):3520-6) (Non-patent Reference 7).

Generally, a recombinant single chain immunotoxin has an intervening sequence between the H chain and the L chain which encodes approximately 15 amino acids (Reiter et al. Recombinant Fv immunotoxins and Fv fragments as novel agents for cancer therapy and diagnosis. Trends Biotechnol. 1998 Dec; 16(12):513-20) (Non-patent Reference 8).

Further, DNA of antigen binding site in H-chain or L-chain and a toxin DNA are linked by genetic manipulation and therewith a protein is produced in E. coli cells while another protein is produced using an L chain or H chain antigen binding site DNA and then a recombinant double-chain immunotoxin is constructed by linking these proteins via S-S bonds (Brinkmann et at. A recombinant immunotoxin containing a disulfide-stabilized Fv fragment. Proc Natl Acad Sci USA. 1993; 90(16):7538-42) (Non-patent Reference 9).

### Chimeric antibodies and humanized antibodies

It has been reported that a chimeric antibody produced in E. coli cells by linking a mouse immunoglobulin antigen binding site (Fab part) DNA and a human-derived immunoglobulin Fc part DNA by genetic manipulation produces only a small amount of antibodies against the mouse antibody part in humans and is useful for clinical administration (Smith et al. Rituximab (monoclonal anti-CD20 antibody): mechanisms of action and resistance. Oncogene. 2003; 22(47):7359-68) (Non-patent Reference 10).

Further, It has been reported that a humanized antibody in which CDR1, CDR2, and CDR3 of a human immunoglobulin is replaced by CDR1, CDR2, and CDR3 of a mouse Fab part produces only a small amount of antibodies against the mouse antibody part and is useful for clinical administration (Kipriyanov. Generation and production of engineered antibodies. Mol Biotechnol. 2004; 26(1):39-60) (Non-patent Reference 11). Liposomes

Administration of liposomes in which a drug is encapsulated with a lipid membrane has been attempted as drug delivery system. Further, in order to deliver a drug to a specific cell, an antibody which specifically binds to the cell can also be contained in the liposome in addition to the drug (Gabizon et al. Targeting folate receptor which folate linked to extremities of poly(ethylene glycol)-grafted liposomes: in vitro studies. Bioconjug Chem. 1999; 10(2):289-98) (Non-patent Reference 12).

It is assumed that a drug delivery system with folate receptor beta (FR-β) is as useful as that with folate receptor alpha (FR-α) and in vitro studies on folate liposomes have been carried out using toxins such as momordin and saporin and anti-cancer agents (Pan XQ et al. Antitumor activity of folate receptor-targeted liposomal doxorubicin in a KB oral carcinoma murine xenograft model. Pharm Res. 2003 Mar; 20(3):417-22 (Non-patent Reference 13); Sudimack et al. Targeted drug delivery via the folate receptor. Adv Drug Deliv Rev. 2000 Mar 30; 41(2):147-62 (Non-patent Reference 14)).

### Rheumatoid arthritis

The present inventors have reported that expression of folate receptor beta (FR-β) is augmented in activated macrophages and synovial macrophages from rheumatoid arthritis patients (Nakashima-Matsushita et al. Selective expression of folate receptor beta and its possible role in methotrexate transport in synovial macrophages from patients with rheumatoid arthritis. Arthritis Rheum. 1999; 42(8):1609-16) (Non-patent Reference 15).

As a mechanism of action of gold agents and methotrexate which are effective in treating RA synovitis, their action of suppressing migration and activation of monocytes and macrophages has been reported (Yamashita et al. Effects of chrisotherapeutic gold compounds on prostaglandin E2 production. Curr Drug Targets Inflamm Allergy. 2003 Sep; 2(3):216-23 (Non-patent Reference 16); Bondeson J. The mechanisms of action of disease-modifying antirheumatic drugs: a review with emphasis on macrophage signal transduction and the induction of proinflammatory cytokines. Gen Pharmacol 1997 Aug; 29(2):127-50 (Non-patent Reference 17)).

Recently, it has been reported that an anti-TNF-α antibody therapy is markedly effective on RA, and the antibody-dependent cytotoxicity via TNF-α on the surface of the macrophage cell membrane has been suggested as a mechanism of its action (Maini RN et al. How does infliximab work in rheumatoid arthritis? Arthritis Res. 2002; 4 Suppl 2:S22-8. Epub 2002 Mar 27) (Non-patent Reference 18).

Further, in experimental arthritis in rats, folate uptake into arthritic areas increases and this uptake has been suggested to be by folate receptor beta (FR-β) expressing cells (Turk et al. Folate-target imaging of activated macrophages in rats with adjuvant-induced arthritis. Arthritis Rheum 2002 Jul; 46(7):1947-55 (Non-patent Reference 19); Paulos CM et al. Folate receptor-mediated targeting of therapeutic and imaging agents to activated macrophages in rheumatoid arthritis. Adv Drug Deli Rev 2004 Apr; 56(8):1205-17 (Non-patent Reference 20)).

The present inventors have reported that an antifolate Ly309887 specific to folate receptor beta (FR-β) suppresses experimental arthritis in mice (Nagayoshi et al. Ly309887, antifolate via the folate receptor suppresses murine type II collagen induced arthritis. Clin Exp Rheumatol. 2003 Nov-Dec; 21(6):719-25) (Non-patent Reference 21).

An example of an immunotoxin which has been administered to human subjects aiming to treat RA is IL-2 denileukin diftitox (Strand V et al. Differential patterns of response in patients with rheumatoid arthritis following administration of an anti-CD5 immunoconjugate. Clin Exp Rheumatol. 1993 Suppl 8:S161-3) (Non-patent Reference 22).

An anti-CD5 antibody ricin A has been reported (Fishwild et al. Administration of an anti-CD5 immunoconjugate to patients with rheumatoid arthritis: effect on peripheral blood mononuclear cells and in vitro immune function. J Rheumatol. 1994; 21(4):596-604) (Non-patent Reference 23).

Further, use of an anti-CD64 antibody ricin A to damage macrophages present in articular cavities has been reported (van Roon JA et al. Selective elimination of synovial inflammatory macrophages in rheumatoid arthritis by an Fc gamma receptor I-directed immunotoxin. Arthritis Rheum. 2003; 48(5):1229-38) (Non-patent Reference 24).

Furthermore, U.S. Patent No. 6,645,495 (Patent Reference 31) describes the construction of anti-CD40L antibody bouganin in claim 7 and its effect in suppressing the growth of activated T cells in Example 4.

U.S. Patent No. 6,346,248 (Patent Reference 32) describes application of anti-CD80 antibody gelonin and anti-CD86 antibody gelonin to autoimmune diseases in claim 2.

### Macrophage activation syndrome

In macrophage activation syndrome, the major pathological condition is considered to be abnormal activation of macrophages (Ravelli et al. Macrophage activation syndrome. Curr Opin Rheumatol. 2002 S; 14(5):548-52) (Non-patent Reference 25).

### Septic shock

Septic shock has generally been recognized as a result of gram-negative bacterial infection; however, today it is revealed that it can also be eventually caused by gram-positive microorganisms, fungi, viruses, and parasites. Microorganisms themselves, their components, or their products induce host cells, particularly macrophages, to release an inflammatory substance such as TNF-α, which triggers a cascade leading to cachexia and septic shock (Evans et al. The role of macrophages in septic shock. Immunobiology. 1996 Oct; 195(4-5):655-9) (Non-patent Reference 26).

### Acute myeloid leukemia

It has been reported that folate receptor beta (FR-β) is rarely expressed in normal cells but the FR-β expression is accelerated in a part of the cells in acute myeloid leukemia (Reddy et al. Expression and functional characterization of the beta-isoform of the folate receptor on CD34(+) cells. Blood. 1999 Jun 1; 93(11):3940-8) (Non-patent Reference 27).

European Patent relating WO 03/072091 (Patent Reference 33) describes in claim 1 FR-β expression of myeloid leukemia cells accelerated by retinoic acid and administered by a liposome which contains folate and a drug, and its specification describes that FR-β expression is observed in 70% of acute myeloid leukemia and that a composition in which a drug is added to a folate liposome suppresses the growth of myeloid leukemia cells in vitro. As an immunotoxin for the treatment of acute myeloid leukemia, humanized anti-CD33 antibody calicheamicin has been approved by FDA in 2000 and has shown a good therapeutic effect (Giles et al. Gemtuzumab ozogamicin in the treatment of acute myeloid leukemia. Cancer. 2003 Nov 15; 98(10):2095-104) (Non-patent Reference 28).

Furthermore, anti-CD30 antibody dianthin conjugate has been reported (Bolognesi et al. Anti-CD30 immunotoxins with native and recombinant dianthin 30. Cancer Immunol Immunother, 1995; 40(2):109-14) (Non-patent Reference 29).

Anti-CD33 antibody gelonin conjugate has been reported ( Xu et al. Antileukemic activity of recombinant humanized M195-gelonin immunotoxin in nude mice. Leukemia. 1996; 10(2):321-6) (Non-patent Reference 30).

Anti-CD33 ricin conjugate has been reported (Russa et al. Effects of anti-CD33 blocked ricin immunotoxin on the capacity of CD34+ human marrow cells to establish in vitro hematopoiesis in long-term marrow cultures. Exp Hematol. 1992; 20(4):442-8) (Non-patent Reference 31).

Anti-CD64 antibody PE conjugate has been reported (Tur et al. Recombinant CD64-specific single chain immunotoxin exhibits specific cytotoxicity against acute myeloid leukemia cells. Cancer Res. 2003; 63(23):8414-9)(Non-patent Reference 32).

Anti-CD64 antibody ricin conjugate has been reported (Zhong et al. Cytotoxicity of anti-CD64-ricin a chain immunotoxin against human acute myeloid leukemia cells in vitro and in SCID mice. J Hematother Stem Cell Res. 2001; 10(1):95-105)(Non-patent Reference 33).

Anti-HIM6 antibody cytosine arabinoside conjugate has been reported (Wang et al. [Studies of two conjugates of monoclonal antibody (HIM6) and cytosine arabinoside] Zhongguo Yi Xue Ke Xue Yuan Xue Bao. 1993; 15(4):286-90) (Non-patent Reference 34).

GM-CSF PE conjugate has been reported (O'Brien et al. A recombinant GM-CSF-PE40 ligand toxin is functionally active but not cytotoxic to cells. Immunol Cell Biol. 1997; 75(3):289-94) (Non-patent Reference 35).

GM-CSF diphtheria toxin conjugate has been reported (Hall et al. DT388-GM-CSF, a novel fusion toxin consisting of a truncated diphtheria toxin fused to human granulocyte-macrophage colony-stimulating factor, prolongs host survival in a SCID mouse model of acute myeloid leukemia. Leukemia. 1999; 13(4):629-33) (Non-patent Reference 36).

IL-3 diphtheria toxin conjugate has been reported (Black et al. Diphtheria toxin-interleukin-3 fusion protein (DT(388)IL3) prolongs disease-free survival of leukemic immunocompromised mice. Leukemia. 2003; 17(1):155-9) (Non-patent Reference 37).

IL-9 PE conjugate and its in vitro and ex vivo effects have been reported (Klimka et al. A deletion mutant of Pseudomonas exotoxin-A fused to recombinant human interleukin-9 (rhIL-9-ETA') shows specific cytotoxicity against IL-9-receptor-expressing cell lines. Cytokines Mol Ther. 1996; 2(3):139-46) (Non-patent Reference 38). References
(1) U.S. Patent No. 6,703,488 (Patent Reference 1)
(2) U.S. Patent No. 6,703,020 (Patent Reference 2)
(3) U.S. Patent No. 6,696,064 (Patent Reference 3)
(4) U.S. Patent No. 6,689,869 (Patent Reference 4)
(5) U.S. Patent No. 6,417,337 (Patent Reference 5)
(6) U.S. Patent No. 6,395,276 (Patent Reference 6)
(7) U.S. Patent No. 6,348,581 (Patent Reference 7)
(8) U.S. Patent No. 6,346,248 (Patent Reference 8)
(9) U.S. Patent No. 6,319,891 (Patent Reference 9)
(10) U.S. Patent No. 6,312,694 (Patent Reference 10)
(11) U.S. Patent No. 6,287,562 (Patent Reference 11)
(12) U.S. Patent No. 6,267,960 (Patent Reference 12)
(13) U.S. Patent No. 6,074,644 (Patent Reference 13)
(14) U.S. Patent No. 6,033,876 (Patent Reference 14)
(15) U.S. Patent No. 5,980,895 (Patent Reference 15)
(16) U.S. Patent No. 5,840,854 (Patent Reference 16)
(17) U.S. Patent No. 5,817,313 (Patent Reference 17)
(18) U.S. Patent No. 5,776,427 (Patent Reference 18)
(19) U.S. Patent No. 5,759,546 (Patent Reference 19)
(20) U.S. Patent No. 5,506,343 (Patent Reference 20)
(21) U.S. Patent No. 5,045,451 (Patent Reference 21)
(22) U.S. Patent No. 4,806,494 (Patent Reference 22)
(23) U.S. Patent No. 4,545,985 (Patent Reference 23)
(24) European Patent relating W0 99/64073 (Patent Reference 24)
(25) European Patent relating No. NZ336576 (Patent Reference 25)
(26) European Patent relating No. CN1330081 (Patent Reference 26)
(27) European Patent relating W0 97/13529 (Patent Reference 27)
(28) European Patent relating WO 98/41641 (Patent Reference 28)
(29) European Patent relating WO 94/13316 (Patent Reference 29)
(30) European Patent relating EP 0583794 (Patent Reference 30)
(31) U.S. Patent No. 6,645,495 (Patent Reference 31)
(32) U.S. Patent No. 6,346,248 (Patent Reference 32)
(33) European Patent relating W0 03/072091 (Patent Reference 33)
(34) Kohler et al. Continuous cultures of fused cells secreting antibody of predefined specificity. Nature. 1975 Aug 7; 256(5517):495-7 (Non-patent Reference 1)
(35) Ogata et al. Cell-mediated cleavage of Pseudomonas exotoxin between Arg279 and Gly280 generates the enzymatically active fragment which translocates to the cytosol. J Biol Chem. 1992; 267(35):25396-401 (Non-patent Reference 2)
(36) Kreitman. Chimeric fusion proteins-Pseudomonas exotoxin-based. Curr Opin Investig Drugs. 2001 2(9):1282-93 (Non-patent Reference 3)
(37) Pastan I. Immunotoxins containing Pseudomonas exotoxin A: a short history. Cancer Immunol Immunother. 2003; 52(5):338-41 (Non-patent Reference 4)
(38) Trail et al. Monoclonal antibody drug immunoconjugates for targeted treatment of cancer. Cancer Immunol Immunother. 2003 May; 52(5):328-37 (Non-patent Reference 5)
(39) Milenic DE. Monoclonal antibody-based therapy strategies: providing options for the cancer patient. Curr Pharm Des. 2002; 8(19):1749-64 (Non-patent Reference 6)
(40) Haasan R et al. Antitumor activity of SS(dsFv)PE38 and SS1(dsFv)PE38, recombinant antimesothelin immunotoxins against human gynecologic cancers grown in organotypic culture in vitro. Clin Cancer Res. 2002 Nov; 8(11):3520-6 (Non-patent Reference 7)
(41) Reiter et al. Recombinant Fv immunotoxins and Fv fragments as novel agents for cancer therapy and diagnosis. Trends Biotechnol. 1998 Dec; 16(12):513-20 (Non-patent Reference 8)
(42) Brinkmann et al. A recombinant immunotoxin containing a disulfide-stabilized Fv fragment. Proc Natl Acad Sci USA. 1993; 90(16):7538-42 (Non-patent Reference 9)
(43) Smith et al. Rituximab (monoclonal anti-CD20 antibody): mechanisms of action and resistance. Oncogene. 2003; 22(47):7359-68 (Non-patent Reference 10)
(44) Kipriyanov. Generation and production of engineered antibodies. Mol Biotechnol. 2004; 26(1):39-60 (Non-patent Reference 11)
(45) Gabizon et al. Targeting folate receptor with folate linked to extremities of poly(ethylene glycol)-grafted liposomes: in vitro studies. Bioconjug Chem. 1999; 10(2):289-98 (Non-patent Reference 12)
(46)Pan XQ et al. Antitumor activity of folate receptor-targeted liposomal doxorubicin in a KB oral carcinoma murine xenograft model. Pharm Res. 2003 Mar; 20(3):417-22 (Non-patent Reference 13)
(47) Sudimack et al. Targeted drug delivery via the folate receptor. Adv Drug Deliv Rev. 2000 Mar 30; 41(2):147-62.(Non-patent Reference 14)
(48) Nakashima-Matsushita et al. Selective expression of folate receptor beta and its possible role in methotrexate transport in synovial macrophages from patients with rheumatoid arthritis. Arthritis Rheum. 1999; 42(8):1609-16 (Non-patent Reference 15)
(49) Yamashita et al. Effects of chrisotherapeutic gold compounds on prostaglandin E2 production. Curr Drug Targets Inflamm Allergy. 2003 Sep; 2(3):216-23 (Non-patent Reference 16)
(50) Bondeson J. The mechanisms of action of disease-modifying antirheumatic drugs: a review with emphasis on macrophage signal transduction and the induction of proinflammatory cytokines. Gen Pharmacol. 1997 Aug; 29(2):127-50 (Non-patent Reference 17)
(51)Maini RN et al. How does infliximab work in rheumatoid arthritis? Arthritis Res. 2002; 4 Suppl 2:S22-8 (Non-patent Reference 18)
(52) Turk et al. Folate-targeted imaging of activated macrophages in rats with adjuvant-induced arthritis. Arthritis Rheum 2002 Jul. 46(7):1947-55 (Non-patent Reference 19)
(53) Paulos CM et al. Folate receptor-mediated targeting of therapeutic and imaging agents to activated macrophages in rheumatoid arthritis. Adv Drug Deli Rev 2004 Apr; 56(8): 1205-17 (Non-patent Reference 20)
(54) Nagayoshi et al. Ly309887, antifolate via the folate receptor suppresses murine type II collagen induced arthritis. Clin Exp Rheumatol 2003 Nov-Dec; 21(6):719-25 (Non-patent Reference 21)
(55) Strand V et al. Differential patterns of response In patients with rheumatoid arthritis following administration of an anti-CD 5 immunoconjugate, Clin Exp Rheumatol. 1993 Suppl 8:S161-3 (Non-patent Reference 22)
(56) Fishwild et al. Administration of an anti-CD5 immunoconjugate to patients with rheumatoid arthritis: effect on peripheral blood mononuclear cells and in vitro immune function. J Rheumatol. 1994; 21(4):596-604 (Non-patent Reference 23)
(57) van Roon JA et al. Selective elimination of synovial inflammatory macrophages in rheumatoid arthritis by an Fc gamma receptor I-directed immunotoxin. Arthritis Rheum. 2003; 48(5):1229-3 (Non-patent Reference 24)
(58) Ravelli et al. Macrophage activation syndrome. Curr Opin Rheumatol. 2002 S; 14(5):548-52 (Non-patent Reference 25)
(59) Evans. The role of macrophages in septic shock. Immunobiology. 1996 Oct; 195(4-5):655-9 (Non-patent Reference 26)
(60) Reddy et al. Expression and functional characterization of the beta-isoform of the folate receptor on CD34(+) cells. Blood. 1999 Jun 1, 93(11):3940-8 (Non-patent Reference 27)
(61) Giles et al. Gemtuzumab ozogamicin in the treatment of acute myeloid leukemia. Cancer. 2003 Nov 15; 98(10):2095-104 (Non-patent Reference 28)
(62) Bolognesi et al. Anti-CD30 immunotoxins with native and recombinant dianthin 30. Cancer Immunol Immunother. 1995; 40(2):109-14 (Non-patent Reference 29)
(63) Xu et al. Antileukemic activity of recombinant humanized M195-gelonin immunotoxin in nude mice. Leukemia. 1996; 10(2):321-6 (Non-patent Reference 30)
(64) Russa et al. Effects of anti-CD33 blocked ricin immunotoxin on the capacity of CD34+ human marrow cells to establish in vitro hematopoiesis in long-term marrow cultures. Exp Hematol, 1992; 20(4):442-8 (Non-patent Reference 31)
(65) Tur et al. Recombinant CD64-specific single chain immunotoxin exhibits specific cytotoxicity against acute myeloid leukemia cells. Cancer Res. 2003; 63(23):8414-9 (Non-patent Reference 32)
(66) Zhong et al. Cytotoxicity of anti-CD64-ricin a chain immunotoxin against human acute myeloid leukemia cells in vitro and in SCID mice. J Hematother Stem Cell Res. 2001; 10(1):95-105 (Non-patent Reference 33)
(67) Wang et al. [Studies of two conjugates of monoclonal antibody (HIM6) and cytosine arabinoside] Zhongguo Yi Xue Ke Xue Yuan Xue Bao. 1993; 15(4):286-90 (Non-patent Reference 34)
(68) O'Brien et al. A recombinant GM-CSF-PE40 ligand toxin is functionally active but not cytotoxic to cells. Immunol Cell Biol. 1997; 75(3):289-94 (Non-patent Reference 35)
(69) Hall et al. GM-CSF Diphtheriatoxin (DT388-GM-CSF, a novel fusion toxin consisting of a truncated diphtheria toxin fused to human granulocyte-macrophage colony-stimulating factor, prolongs host survival in a SCID mouse model of acute myeloid leukemia. Leukemia. 1999; 13(4):629-33 (Non-patent Reference 36)
(70) Black et al. Diphtheria toxin-interleukin-3 fusion protein (DT(388)IL3) prolongs disease-free survival of leukemic immunocompromised mice. Leukemia. 2003; 17(1):155-9 (Non-patent Reference 37)
(71) Klimka et al. A deletion mutant of Pseudomonas exotoxin-A fused to recombinant human interleukin-9 (rhIL-9-ETA') shows specific cytotoxicity against IL-9-receptor-expressing cell lines. Cytokines Mol Ther. 1996; 2(3):139-46 (Non-patent Reference 38)

### SUMMARY OF THE INVENTION

However, there has been no report on the construction of an IgG-type FR-β monoclonal antibody effectively applying Non-patent Reference 1. Further, there has been no report on the construction of an immunotoxin which is a conjugate of genetically engineered PE with an FR-β monoclonal antibody by effectively applying Non-patent Reference 2 and Non-patent Reference 3. Accordingly, an objective of the present invention is to construct an FR-β monoclonal antibody PE conjugate.

Further, there has been no report on the construction of an immunotoxin which is a conjugate of a toxin other than PE with an FR-β monoclonal antibody by effectively applying Non-patent Reference 4, Non-patent Reference 5 and Non-patent Reference 6 and accordingly an objective of the present invention is to construct an FR-β monoclonal antibody immunotoxin.

To date, a number of immunotoxins with use of recombinant PEs have been disclosed and their effectiveness in various diseases has been shown in vitro and in vivo. However, immunotoxins described in Patent References 1, 2, 3, 4, 5, 6, 7, 8, 9, 10, 11, 12, 13, 14, 15, 16, 17, 18, 19, 20, 21, 22, 23, 24, 25, 26, 27, 28, 29, and 30 are not immunotoxins targeting activated macrophages only and have a problem that no satisfactory effect can be obtained in treating diseases in which activated macrophage is the major pathological condition and accordingly an objective of the present invention is to construct an FR-β monoclonal antibody immunotoxin which is effective in treating diseases in which activated macrophage is the major pathological condition.

A recombinant single chain immunotoxin and a recombinant double chain immunotoxin can be constructed by linking a DNA of an antigen binding site of the H chain or L chain of an antibody with a DNA of a toxin by genetic manipulation and therewith producing a protein in E. coli cells. A recombinant immunotoxin has advantages such that it can easily enter inside cells because of its small molecular weight and that its mass purification is more possible than the chemical construction of antibody-toxin conjugates.

To date, since genetic sequences of the H chain and the L chain of an FR-β monoclonal antibody have not been elucidated, there has been no report on the construction of a FR-β monoclonal antibody recombinant single-chain immunotoxin by effectively applying Non-patent Reference 7 and Non-patent Reference 8 and the construction of a recombinant FR-β monoclonal antibody double-chain immunotoxin by effectively applying Non-patent Reference 9 and accordingly an objective of the present invention is to determine the genetic sequences of the H chain and the L chain of an FR-β monoclonal antibody for the construction of a recombinant FR-β monoclonal antibody single-chain immunotoxin and a recombinant FR-β monoclonal antibody double-chain immunotoxin.

It has been described that a chimeric antibody produces a smaller amount of antibodies against a mouse antibody part in humans and is useful in clinical administration. To date, since genetic sequences of the H chain and the L chain of an FR-β monoclonal antibody have not been elucidated, there has been no report on the construction of a chimeric antibody by effectively applying Non-patent Reference 10 and accordingly, an objective of the present invention is to determine the genetic sequences of the H chain and the L chain of an FR-β monoclonal antibody for the construction of a chimeric antibody of the FR-β monoclonal antibody.

Further, it has been described that a humanized antibody in which CDR1, CDR2, and CDR3 of a human immunoglobulin are replaced with CDR1, CDR2, and CDR3 of the mouse Fab part produces a small amount of antibodies against the mouse antibody part and is useful in clinical administration.

To date, since genetic sequences of the H chain and the L chain of an FR-β monoclonal antibody have not been elucidated, there has been no report on the construction of a humanized antibody by effectively applying Non-patent Reference 11 and accordingly an objective of the present invention is to determine genetic sequences of the H chain and the L chain of an FR-β monoclonal antibody for the construction of the humanized FR-β monoclonal antibody.

For drug delivery to a specific cell, use of an antibody, which binds to the specific cell, added into a liposome in addition to a drug is useful as a therapeutic method. However, there has been no report on the use of an FR-β monoclonal antibody for the construction of a liposome by effectively applying Non-patent Reference 12, Non-patent Reference 13, and Non-patent Reference 14 and accordingly an objective of the present invention is to construct an FR-β monoclonal antibody for the construction of a liposome containing the FR-β monoclonal antibody.

The role of activated macrophages in the pathological condition of rheumatoid arthritis is known and effectiveness of the therapies for the purpose of regulating macrophage activation has been reported in Non-patent References 15, 16, 17, and 18. However, these therapies are not with an immunotoxin and have problems in terms of capability in killing and elimination of the cells.

Further, in Non-patent Reference 19 and Non-patent Reference 20, use of a conjugate of a folate with an isotope is described but the problem thereof is that there is no mention on a therapeutic effect of this conjugate. An objective of the present invention is to suppress activated macrophages in rheumatoid arthritis by an FR-β monoclonal antibody conjugate. Further, Non-patent Reference 21 by the present inventors is a report showing that a drug binding to folate receptor beta (FR-β) is effective in arthritis mice but is not a report with use of an FR-β monoclonal antibody conjugate and accordingly an objective of the present invention is to suppress activated macrophages in rheumatoid arthritis by an FR-β monoclonal antibody conjugate.

To date, immunotoxins for the purpose of treating rheumatoid arthritis have been reported in Non-patent References 22, 23, 24, 31, and 32. However, lymphocytes and non-activated macrophages are also included as a target for the action and the suppression is not solely on activated macrophages, which disadvantageously causes various side effects. Further, toxins other than PE are used as a toxin and thus the action of PE as a toxin is not clear. An objective of the present invention is to suppress activated macrophages in rheumatoid arthritis by an FR-β monoclonal antibody immunotoxin, in particular an FR-β monoclonal antibody PE conjugate.

Non-patent Reference 25 has reported that abnormal activation of macrophages is the major pathological condition in macrophage activation syndrome and thus death or elimination of the activated macrophages is desirable. However, there is no mention about immunotoxins as a therapeutic means in Non-patent Reference 25. An objective of the present invention is to treat septic shock with an FR-β monoclonal antibody toxin conjugate.

Non-patent Reference 26 has reported that abnormal activation of macrophages is the major pathological condition in septic shock and thus death or elimination of the activated macrophages is desirable. However, there is no mention about immunotoxins as a therapeutic means in Non-patent Reference 26. An objective of the present invention is to treat septic shock with an FR-β monoclonal antibody immunotoxin.

It has been reported that the expression of folate receptor beta (FR-β) is increased in some acute myeloid leukemia, and Non-patent Reference 27 and Patent Reference 33 have reported that a liposome containing folate and a drug suppresses the growth of acute myeloid leukemia cells; however, this liposome treatment is not specific to FR-β expressing cells since folate receptors also include FR-α, which disadvantageously causes various side effects. Further, drug resistance is known to occur in leukemia cells and combined use of drugs is desirable. An objective of the present invention is to treat acute myeloid leukemia, specific to FR-β expressing acute myeloid leukemia cells, using a liposome containing an FR-β monoclonal antibody. Effects of immunotoxins in treating acute myeloid leukemia have been reported in Non-patent References 28, 29, 30, 31, 32, 33, 34, 35, 36, 37, and 38. However, monoclonal antibodies or cytokines used as a ligand are not specifically bound to acute myeloid leukemia cells and no FR-β monoclonal antibody is used in any cases, which disadvantageously causes various side effects. Further, disappearance of surface antigens and induction of drug resistance have been known to occur in leukemia cells and combined use of drugs Is desirable.

The present invention provides an FR-β monoclonal antibody, in particular an IgG-type FR-β monoclonal antibody, for the treatment of acute myeloid leukemia.

An objective of the present invention is to treat acute myeloid leukemia, specific to an FR-β expressing acute myeloid leukemia cells, by an anti-FR-β monoclonal antibody immunotoxin conjugate.

The present invention is based on the finding that a new substance which damages activated macrophage cells and acute myeloid leukemia cells without acting on monocytes as macrophage precursor cells and non-activated macrophages is found and the present invention provides a therapeutic agent that is useful in treating diseases which cannot be satisfactorily treated with conventional therapeutic agents, utilizing a novel action mechanism of the substance and in combination with conventionai therapeutic agents to further increase its therapeutic effect.

An object of the present invention is to provide a immunotoxin comprising a toxin molecule, and a monoclonal antibody which is capable of binding to a human FR-β antigen present on the cell surface of activated macrophages and leukemia cells and induces cell death by binding to the FR-β expressing cells.

Another object of the present invention is to provide a method of treating a disease in which macrophage activation is the major pathological condition, such as rheumatoid arthritis, juvenile rheumatoid arthritis, macrophage activation syndrome, and septic shock, by binding of the abovementioned immunotoxin against an FR-β antigen, resulting in eliminating FR-β expressing cells and suppressing local inflammatory response. This method comprises administering an immunotoxin capable of binding to a human FR-β antigen on the surface of an activated macrophage cell in a therapeutically effective amount together with a pharmaceutically acceptable excipient to a patient who needs treatment.

A further object of the present invention is to provide a method of treating leukemia by binding of the abovementioned immunotoxin against an FR-β antigen and eliminating FR-β expressing tumor cells. This method comprises administering an immunotoxin capable of binding to FR-β on the surface of a tumor cell derived from a myelocyte in a therapeutically effective amount together with a pharmaceutically acceptable excipient to a patient who needs treatment.

Yet another object of the present invention is to provide a finding on the genetic sequence of an FR-β monoclonal antibody for the construction of a recombinant immunotoxin, a chimeric antibody, and a humanized antibody of the FR-β monoclonal antibody.

The folate receptor beta (FR-β) cannot be expressed in other than myeloid cells and the expression is low in cells of healthy humans. Therefore, a therapeutic method applying an antibody against the FR-β antigen is considered to be useful; however, there has been no report on the construction of an IgG-type anti-FR-β monoclonal antibody with a high affinity to the FR-β antigen. To date, there has been no suggestion to use an FR-β monoclonal antibody for eliminating FR-β expressing activated macrophages in a disease in which macrophage activation is the major pathological condition. Further, there has been no suggestion to use the FR-β monoclonal antibody for eliminating FR-β expressing leukemia cells in leukemia.

Since folate receptor beta (FR-β) is expressed only in activated macrophages or acute myeloid leukemia cells but not in monocytes as macrophage precursor cells and non-activated macrophages, the present inventors have considered that it is an effective therapeutic method to kill or eliminate activated macrophages or acute myeloid leukemia cells by utilizing an FR-β monoclonal antibody for the treatment of a disease in which FR-β expressing cells are involved in the pathological condition. As a result of intensive research effort, the present inventors have newly constructed a monoclonal antibody against folate receptor beta (FR-β) specific to activated macrophages and thus completed the invention to accomplish the abovementioned objectives by binding this antibody to a toxin to construct an immunotoxin.

Namely, the present invention is based on the finding that a conjugate of an antibody against an FR-β antigen with a toxin (immunotoxin) effectively kills cells which express FR-β molecules. The present invention is from the thought that, FR-β monoclonal antibody immunotoxin is useful to prevent or treat diseases or symptoms which are mediated by cells expressing folate receptor beta (FR-β). Further, the present invention is based on the finding that, as effective component, an immunotoxin having an FR-β monoclonal immunotoxin, in particular, a conjugate of an IgG-type monoclonal antibody with a genetically engineered Pseudomonas aeruginosa toxin is cytotoxic to activated macrophage cells at a low concentration, and an action mechanism to inhibit the growth of folate receptor beta-expressing myeloid leukemia cells. Thus, the present invention has realized a novel therapeutic agent for the treatment of a disease wherein macrophages have a major role in its pathological condition or leukemia.

The term "antibody" as used in this specification includes polyclonal antibodies, monoclonal antibodies, humanized antibodies, single chain antibodies, fragments of these antibodies such as Fab fragments, F(ab)'₂ fragments, and Fv fragments, and other fragments which maintain an antigen-binding capacity of the parent antibodies.

The term "monoclonal antibody" as used in this specification means an antibody group consisting of a single antibody population. This term does not intend to limit in terms of the kind or origin of the antibody and the method of the production of the antibody. This term includes complete immunoglobulins as well as Fab fragments, F(ab)₂ fragments, Fv fragments and other fragments which maintain the antigen-binding capacity of the antibodies. Mammalian and avian monoclonal antibodies can also be used in this invention.

The term "single chain antibody" used in this specification refers to an antibody which is prepared by determining a binding region (in both the H chain and the L chain) of an antibody having a binding capacity and adding a binding site so as to maintain the binding capacity. In this way, a thoroughly simplified antibody substantially having solely a variable region site necessary for binding to antigen is formed. The term "double chain antibody" used in this specification refers to an antibody which is prepared by determining a binding region (in both the H chain and the L chain) of an antibody having a binding capacity and linking the H chain or the L chain to the L chain or the H chain via s-s bonds. In this way, a thoroughly simplified antibody substantially having solely a variable region site necessary for binding to antigen is formed.

In the present invention, "immunotoxin (IT)" refers to a chimeric molecule in which a cell binding ligand is bound to a toxin or its subunit. The toxin part of the immunotoxin is derived from various origins such as plants and bacteria and a toxin derived from humans and a synthetic toxin (drug) can also be used.

Preferably, the toxin part is derived from a plant toxin, such as type-1 or type-2 ribosome inactivated protein (RIP). The type-2 ribosome inactivated protein includes, for example, ricin. The type-1 RIP is particularly suitable to construct an immunotoxin according to the present invention. Examples of the type-1 RIP include bacterial toxins such as Pseudomonas exotoxin (PE) and diphtheria toxin. Other usable toxins are bryodin, momordin, gelonin, saporin, bouganin and the like.

The ligand part of IT generally refers to a monoclonal antibody which binds to a selected target cell. The IT part to be used in the present invention is a bacterial toxin, Pseudomonas exotoxin (PE). Specifically, the toxin has an ADP-ribosylation activity and translocation activity through the cell membrane. More specifically, PE becomes an active form when its amino acid sequence is cleaved between positions 279 and 280 and can be constructed by transforming E. coli with an expression plasmid containing a DNA encoding PE which lacks a natural toxin receptor binding domain Ia.

A PE binding recombinant immunotoxin of the present invention lacks an Ia domain to bind to the cell surface, starts from position 280 of the amino acid sequence, and has an addition of KDEL and REDLK at the C-terminal site to increase its cytotoxicity. Specifically, nonspecific toxicity is markedly decreased since the toxin has no cell binding activity. More specifically, a genetically engineered PE has a lower toxicity to human or animal cells in vitro and shows a lower toxicity to the liver when administered in vivo than non-engineered PE.

Further, the term "recombinant single chain immunotoxin" as used in the present invention refers to a protein which is constructed by linking a DNA of an antigen binding site of the H chain or the L chain of an antibody with a DNA of a toxin by genetic manipulation and therewith producing a protein in E. coli cells. Specifically, a recombinant single chain immunotoxin generaiiy includes a intervening sequence between the H chain and the L chain which is translated in about 15 amino acids (Reiter et al. Recombinant Fv immunotoxins and Fv fragments as novel agents for cancer therapy and diagnosis. Trends Biotechnol. 1998 Dec; 16(12):513-20).

The term "recombinant double-chain immunotoxin" as used in the present invention refers to a protein which is constructed by linking a DNA of an antigen binding site of the H chain or the L chain of an antibody with a DNA of a toxin by genetic manipulation, producing a protein in E. coli cells, separately producing a protein using the L chain or H chain of antigen binding site DNA, and linking these two proteins via S-S bonds (Brinkmann et al. A recombinant immunotoxin containing a disulfide-stabilized Fv fragment. Proc Natl Acad Sci USA. 1993, 90(16):7538-42).

The term "chimeric antibody" as used in the present invention refers to an antibody which is constructed by linking a DNA of a mouse immunoglobulin antigen binding site (Fab part) with a DNA of a human-derived immunoglobulin Fc site by genetic manipulation and producing a protein in E. coli cells (Smith et al. Rituximab (monoclonal anti-CD20 antibody): mechanisms of action and resistance. Oncogene. 2003; 22(47):7359-68).

The term "humanized antibody" as used in the present invention refers to an antibody in which CDR1, CDR2, and CDR3 of a human immunoglobulin are replaced with CDR1, CDR2, and CDR3 of a mouse Fab part (Kipriyanov. Generation and production of engineered antibodies. Mol Biotechnol. 2004; 26(1):39-60).

The term "liposome" as used in the present invention refers to a structure composed of a lipid membrane which encapsulates a drug as a drug delivery system. Specifically, it refers to a liposome containing an antibody which binds to a specific cell in addition to a drug in order to deliver the drug to the specific cell ( Gabizon et al. Targeting folate receptor with folate linked to extremities of poly(ethylene glycol)-grafted liposomes: in vitro studies. Bioconjug Chem. 1999; 10(2):289-98).

Examples of biologically and chemically active enzymes as used in the present invention include enzymes acting on the coagulation system, such as urokinase, plasmin, plasminogen, staphylokinase, and thrombin and proteolytic enzymes, such as metalloprotease, collagenase, gelatinase, and stromelysin.

Examples of cytokines used in the present invention include those which have antitumor activity, such as interferon, TGF-β, and TNF-α, endostatin which inhibits angiogenesis, and those which have anti-inflammatory activity, such as IL-1 receptor antagonists, IL-4, IL-10, IL-19, IL-20, IL-22, IL-24, IL-26, IL-28, and IL-29.

Examples of isotopes as used in the present invention include galium-67, galium-68, indium-111, indium-113, iodine-123, iodine-125, iodine-131, technetium-99, yttrium-90, rubidium-97, and rubidium-103.

In the present invention, "chemotherapeutic agent" refers to a molecule having a cytotoxic activity. Specific examples of the agent include metabolic antagonists such as cytosine arabinoside, fluorouracil, methotrexate, aminopterin, anthracycline, mitomycin, demecolcine, etoposide, and mithramycin; alkylating agents such as chlorambucil, melpharan, and endoxan; DNA synthesis inhibitors such as daunorubicin, doxorubicin, and adriamycin; and tubulin inhibitors such as colchicine, taxane, and vinca alkaloids including vinblastine and vincristine.

In the present invention, "folate receptor beta (FR-β)" refers to a surface antigen which is expressed in activated macrophages and acute myeloid leukemia cells and is a molecule involved in intracellular transportation of folate. In the present invention, "rheumatoid arthritis (RA)" refers to a chronic inflammatory disease which has symptoms such as multiple joint swelling and pain and is characterized by joint bone destruction, in which macrophage-like cells present in the RA synovial membrane produce cytokines such as IL-1B, IL-6, IL-8, IL-10, IL-15, MCP-1, MIP-1A, TNF-A, M-CSF, GM-CSF, TGF-β, VEGF, PDGF, IL-1 receptor antagonists which antagonize with IL-1, NO, active oxygen, various cathepsins, and various metalloproteases.

In the present invention, "juvenile rheumatoid arthritis (JRA)" refers to a cause-unknown disease which occurs in youngsters of no more than 16 years old and causes chronic joint inflammation as a major symptom associated with various non-joint symptoms. More specifically, JRA is classified into three categories, i.e., systemic, polyarticular and pauciarticular types. The systemic type causes remittent fever from normal temperature to 40°C, rash, systemic lymph node swelling, liver/spleen swelling, pericarditis, pleuritis, and the like; the polyarticular type is often associated with subcutaneous nodules and causes systemic symptoms such as fever and fatigue, insufficient growth and weight loss; and the pauciarticular type causes iritis and occasionally weakening or loss of eyesight.

In the present invention, "macrophage activation syndrome" refers to a pathological condition which exhibits fever, pancytopenia, disorder of hepatic functions, disseminated intravascular coagulation, and blood cell phagocytosis in the bone marrow. Specifically, it causes hypercytokinemia, especially with a high TNF-α value and macrophage activation is its major pathological condition (Ravelli et al. Macrophage activation syndrome. Curr Opin Rheumatol. 2002 S; 14(5):548-52).

As used in the present invention, "septic shock" is generally recognized as a result of gram-negative bacterial infection; however, today it is evident that it can also be caused as a result of infection with gram-positive microorganisms and probably with fungi, viruses and parasites.

In the present invention, "acute myeloid leukemia" refers to an abnormal growth of myeloid cells and causes death from infection and bleeding in untreatable cases. Specifically, it is acute myeloid leukemia in which FR-β is expressed ( Russ et al. Folate receptor type beta is a neutrophilic lineage marker and is differentially expressed in myeloid leukemia. Cancer. 1999 Jan 15, 85(2):348-57).

A subject of the present invention is FR-β monoclonal antibodies. It includes IgG type antibodies. The FR-β monoclonal antibodies of the present invention also include antibodies produced by clone 36 cell obtained by immunizing a mouse with FR-β expressing B300-19 cell and then fusing spleen cells from the mouse with mouse myeloma cells. The FR-β monoclonal antibodies of the present invention also include antibodies produced from clone 94b cell obtained by immunizing a mouse with FR-β expressing B300-19 cell and then fusing spleen cells from the mouse with mouse myeloma cells.

A subject of the present invention is genes of the H chain and the L chain of the FR-β monoclonal antibody clone 36 and proteins encoded by these genes. Further, the present invention also includes variants which have biological activities substantially equivalent to those of these genes or proteins. The present invention also includes humanized FR-β monoclonal antibodies which are obtained by chimerization of the genes of the H chain and the L chain of clone 36.

A subject of the present invention is genes of the H chain and the L chain of FR-β monoclonal antibody clone 94b and proteins encoded by these genes. Further, the present invention also includes variants which have biological activities substantially equivalent to those of these genes or proteins. The present invention also includes humanized FR-β monoclonal antibodies which are obtained by chimerization of the genes of the H chain and the L chain of clone 94b.

An FR-β antibody immunotoxin of the present invention is a conjugate of an FR-β monoclonal antibody with a toxin. Here, the toxin includes, but is not limited to, ricin A chain, deglycosylated ricin A chain, a ribosome inactivating protein, alpha-sarcin, gelonin, aspergillin, restrictocin, ribonuclease, epipodophyllotoxin, diphtheria toxin, and Pseudomonas exotoxin.

The present invention also includes a recombinant FR-β antibody immunotoxin produced using H chain and L chain genes of the clone 36.

The present invention also includes a recombinant FR-β antibody immunotoxin produced using H chain and L chain genes of the clone 94b L chain gene.

The present invention also includes a conjugate of at least one biologically or chemically active molecule selected from the group consisting of enzymes, cytokines, isotopes, and chemotherapeutic agents with an FR-β monoclonal antibody.

The present invention also includes a liposome containing an FR-β monoclonal antibody and a chemotherapeutic agent.

The present invention also includes a pharmaceutical composition containing at least one component selected from said FR-β antibody immunotoxin, said conjugate, and said liposome as an active ingredient.

The present invention also includes a therapeutic agent for treating a disease, in which macrophages are mainly involved in its pathological condition, containing at least one component selected from said FR-β antibody immunotoxin, said conjugate, and said liposome as active ingredient.

The present invention also includes the abovementioned therapeutic agent wherein the disease in which macrophages are mainly involved in its pathological condition is a disease selected from the group consisting of rheumatoid arthritis, juvenile rheumatoid arthritis, macrophage activation syndrome, and septic shock.

The present invention also includes a therapeutic agent for treating rheumatoid arthritis or juvenile rheumatoid arthritis, in which the administration form for the abovementioned therapeutic agent is joint injection.

The present invention also includes a therapeutic agent for treating leukemia containing at least one component selected from said FR-β antibody immunotoxin, said conjugate, and said liposome as an active ingredient.

The present invention also includes the abovementioned therapeutic agent in which the leukemia is acute myeloid leukemia.

The FR-β antibody immunotoxin of the present invention induces apoptosis, a form of programmed cell death, in FR-β expressing macrophages. Further, the FR-β antibody immunotoxin of the present invention acts on FR-β expressing B300-19 cells and induces apoptosis, a form of programmed cell death, in the FR-β expressing B300-19 cells.

As explained above, the present invention constructs an FR-β monoclonal antibody which acts on activated macrophages and acute myeloid leukemia cells but not on macrophage precursor cells such as monocytes and non-activated macrophages, and therewith provides a therapeutic agent that is useful in treating diseases which cannot be satisfactorily treated with conventional therapeutic agents and further increases its therapeutic effect in combination with conventional therapeutic agents.

There is provided a therapeutic agent having a specific therapeutic effect on activated macrophages and acute myeloid leukemia cells by constructing an IgG-type FR-β monoclonal antibody with a low molecular weight and a high affinity to the FR-β antigen.

Gene sequences of variable regions of the H chain and the L chain of IgG-type FR-β monoclonal antibodies clone 36 and clone 94b have been elucidated to make it possible to provide a chimeric antibody, a humanized antibody, and a recombinant antibody. Further, these conjugates provide therapeutic agents which cause only a weak allergic reaction to mouse proteins and can be produced in a large scale.

The base sequence of the gene for the H chain of the antibody clone 36 is represented by SEQ ID NO: 1 of the Sequence Listing. The amino acid sequence of the protein encoded by the sequence is also shown along with the base sequence. The base sequence of the gene for the L chain of clone 36 is represented by SEQ ID NO: 2 of the Sequence Listing along with the amino acid sequence of the protein encoded by this base sequence. The gene sequence of the H chain of clone 94b is represented by SEQ ID NO: 3 of the Sequence Listing along with the amino acid sequence of the protein encoded by this gene sequence. The gene sequence of the L chain of clone 94b is represented by SEQ ID NO: 4 of the Sequence Listing along with the amino acid sequence of the protein encoded by this gene sequence.

In this specification, a gene having a base sequence which comprises partial deletions, substitutions, or additions in the base sequence shown by SEQ ID NO: 1 refers to a gene in which less than 20, preferably less than 10, more preferably less than 5 bases are substituted in the base sequence shown by SEQ ID NO: 1. Further, the base sequence of such gene has a homology of 90% or more, preferably 95% or more, more preferably 99% or more to the base sequence shown by SEQ ID NO: 1. Further, such gene and the gene having the base sequence shown by SEQ ID NO: 1 form a hybrid under stringent conditions. The same is true in modified base sequence relative to the base sequences shown by SEQ ID NO: 2, SEQ ID NO: 3, or SEQ ID NO: 4. Such genes also fall within the scope of the present invention as long as they encode a protein which has biological activities substantially equivalent to the H chain or the L chain of clone 36 or the H chain or the L chain of clone 94b.

By using genetic recombination technology, an artificial mutation can be introduced into a specific site of basic DNA without changing basic characteristics of said DNA or to improve these characteristics. Similarly, a gene having a natural base sequence or a gene having a non-natural base sequence provided by the present invention can be modified to a gene having characteristics equivalent to or improved from those of the natural gene by artificial insertions, deletions and substitutions. The present invention also includes such mutant genes.

Further, in this specification, a protein having an amino acid sequence which comprises partial deletions, substitutions, or additions in the amino acid sequence encoded by the base sequence shown by SEQ ID NO: 1 refers to a protein in which less than 20, preferably less than 10, more preferably less than 5 amino acids are substituted in the amino acid sequence encoded by the base sequence shown by SEQ ID NO: 1 (the amino acid sequence provided along with SEQ ID NO: 1). Further, the amino acid sequence of such a protein has a homology of 95% or more, preferably 97% or more, more preferably 99% or more to the amino acid sequence encoded by the base sequence shown by SEQ ID NO: 1. The same is true in modified amino acid sequence encoded by the base sequence represented by SEQ ID NO: 2, SEQ ID NO: 3, or SEQ ID NO: 4. Such proteins also fall within the scope of the present invention as long as they have biological activities substantially equivalent to the H chain or the L chain of clone 36 or the H chain or L chain of clone 94b.

In the present specification, "substantially equivalent" means that activities of the protein, such as a physiological activity to specifically bind to an FR-β antigen and a biological activity, are substantially the same. It may also includes the case of having the substantially the same quality of activities, such as a capability of specifically binding to an FR-β antigen, or being physiologically, pharmacologically or biologically the same in quality. Further, the activities are preferably the same in quantity. However, the quantity element of the activities may be different.

In the present specification, the "stringent" conditions for hybridization can be appropriately selected by those skilled in the art. Specifically, the hybridization can be carried out, for example, by the following procedure. A DNA or RNA molecule transferred onto a membrane is hybridized with a labeled probe in an appropriate hybridization buffer. The hybridization buffer contains, for example, 5× SSC, 0.1% by weight N-lauroyl sarcocine, 0.02wt% SDS, 2wt% blocking reagent for nucleic acid hybridization, and 50% formamide. The blocking agent for nucleic acid hybridization is prepared, for example, by dissolving a commercial blocking reagent for nucleic acid hybridization into a buffer solution containing 0.1 M maleic acid and 0.15 M sodium chloride (pH 7.5) at a concentration of 10%. The 20x SSC consists of 3 M sodium chloride and 0.3 M citric acid and SSC is preferably used at a concentration of 3× to 6× SSC, more preferably 4× to 5× SSC.

The temperature for hybridization is 40 to 80°C, preferably 50 to 70°C, more preferably 55 to 65°C. After several hours to overnight incubation, the reaction solution is washed with a washing buffer. The washing is carried out preferably at room temperature, more preferably at the temperature for hybridization. The washing buffer contains 6× SSC + 0.1wt% SDS solution, preferably 4× SSC + 0.1wt% SDS solution, more preferably 2× SSC + 0.1wt% SDS solution, furthermore preferably 1× SSC + 0.1wt% SDS solution, and most preferably 0.1× SSC + 0.1wt% SDS solution. The membrane is washed with such a washing buffer and a DNA molecule or an RNA molecule hybridized with the probe can be distinguished using the label used for the probe.

Conventional immunotoxins are not to target activated macrophages only, which causes such problems as side effects and insufficient effects in diseases in which activated macrophages are the major pathological condition. Accordingly, the present invention provides immunotoxins which are effective in diseases in which activated macrophages are the major pathological condition.

In the present invention, a conjugate of an FR-β monoclonal antibody is prepared with at least one selected from enzymes, cytokines, isotopes, and chemotherapeutic agents, which induce specific cell death or elimination of activated macrophages in diseases in which activated macrophages are the major pathological condition. Accordingly, such conjugate provides a novel therapeutic agent.

In order to deliver a drug to a specific cell, encapsulation of an antibody which specifically binds to the cell into a liposome in addition to the drug is useful as a therapeutic method; however, use of an FR-β monoclonal antibody for such purpose has not so far been reported. The present invention provides a liposome which has a novei action mechanism and causes little side effects in diseases in which activated macrophages are the major pathological condition.

According to the present invention, there is provided a therapeutic agent which has a novel action mechanism, causes little side effects and induces specific cell death or elimination of activated macrophages in rheumatoid arthritis, juvenile rheumatoid arthritis, macrophage activation syndrome, and septic shock in which activated macrophages are the major pathological condition, using a conjugate of an FR-β monoclonal antibody with at least one selected from toxins, enzymes, cytokines, isotopes, and chemotherapeutic agents or a liposome containing an FR-β monoclonal antibody.

A conjugate of an FR-β monoclonal antibody with at least one selected from toxins, enzymes, cytokines, isotopes, and chemotherapeutic agents or a liposome containing an FR-β monoclonal antibody obtained according to the present invention can be used as a local joint injection In rheumatoid arthritis and juvenile rheumatoid arthritis. Accordingly the present invention provides a novel therapeutic agent to eliminate local joint inflammation.

Since many of therapeutic agents to treat leukemia also act on normal cells, they cause various side effects. Further, it has been known that disappearance of surface antigens and drug resistance occur in leukemia cells and thus a therapeutic agent with a novel action mechanism has been desired. A conjugate of an FR-β monoclonal antibody with at least one selected from toxins, enzymes, cytokines, isotopes, and chemotherapeutic agents and a liposome containing an FR-β monoclonal antibody obtained according to the present invention exhibit specific cell death or elimination of FR-β expressing leukemia cells and thus provide a novel therapeutic agent having a novel action mechanism with few side effects.

### BRIEF DESCRIPTION OF THE DRAWINGS

Fig. 1 shows the reactivity of an anti-FR-β antibody of the present invention.
Fig. 2 shows the separation of an anti-FR-β antibody immunotoxin conjugate of the present invention and a toxin and the ADP-ribosylation activity of these molecules. IT represents immunotoxin. PE represents Pseudomonas exotoxin.
Fig. 3 shows that an anti-FR-β antibody immunotoxin conjugate of the present invention contains a toxin. IT represents immunotoxin. mAB represents FR-β monoclonal antibody. PE represents Pseudomonas exotoxin.
Fig. 4 shows cell death in B300-19 cells by an FR-β antibody immunotoxin of the present invention. In the drawing, 24h, 36h, and 48h represent the cell death after 24 hours, after 36 hours, and after 48 hours, respectively.
Fig. 5 shows expression of FR-β with an adenovector in macrophages.
Fig. 6 shows cell death in FR-β expressing macrophages by an FR-β antibody immunotoxin of the present invention.
Fig. 7 shows that FR-β is expressed in rheumatoid arthritis synovial cells.
Fig. 8 shows cell death of rheumatoid arthritis synovial cells by an FR-β antibody immunotoxin of the present invention.
Fig. 9 shows an SDS-polyacrylamide electrophoresis pattern for a recombinant double chain Fv anti-FR-β PE chimeric antibody.
Fig. 10 shows cell death in FR-β expressing B300-19 cells by a recombinant double chain Fv anti-FR-β PE antibody at various concentrations.
Fig. 11 shows cell death in FR-β expressing HL-60 cells by a recombinant double chain Fv anti-FR-β PE antibody at various concentrations.

### DETAILED DISCRIPTION OF THE INVENTION

### [Construction of FR-β expressing cells]

The present inventors have constructed an FR-β expressing B300-19 cell by the following method. First, the FR-β gene is incorporated into a pEF-BOS vector. The vector is not limited to the pEF-BOS vector and any mammalian expression vector can be used. Next, the FR-β gene is transfected into a mouse B300-19 cell using the lipofectamine method. The gene transfection method can be the electropolation method. Further, the cell line can be any cell line derived from Balb/C mice.

By immunization using this cell, the present inventors have constructed an IgG-type FR-β monoclonal antibody which exhibits a high affinity to the FR-β antigen and has a low molecular weight, using the cell fusion method. The antibody and a toxin molecule are chemically conjugated by one of various known chemical methods, for example, using a crosslinker having a different divalent binding group, such as SPDP, carbodiimide, and glutaraldehyde. Methods for the production of various immunotoxins are known in the art and are described, for example, in Monoclonal Antibody-Toxin Conjugates: Aiming the Magic Bullet, Thorpe et al. Monoclonal Antibodies in Clinical Medicine, Academic Press, pp. 168-190 (1982) and Waldman, Science, 252:11657 (1991). These two literatures are incorporated herewith by reference.

### [Construction of FR-β antibody immunotoxin]

The present inventors have constructed an immunotoxin by conjugating the abovementioned antibody with a genetically engineered Pseudomonas exotoxin (PE) using succinimidyl trans-4-(maleimidylmethyl)cyclohexane 1-carboxylate (SMCC) by the method of Haasan et al. (Haasan et al. Anti-tumor activity of K1-LysPE38QQR, an immunotoxin targeting mesothelin, a cell-surface antigen overexpressed in ovarian cancer and malignant mesothelioma. J Immunother. 2000 J; 23(4):473-9). Toxins to be used in the present invention include, in addition to PE, ricin A chain, deglycosylated ricin A, ribosome inactivating proteins, α-sarcin, gelonin, aspergillin, restrictocin, ribonuclease, epipodophyllotoxin, diphtheria toxin, and Pseudomonas exotoxin.

The antibody can be fused with a toxin using recombination technology in the same manner as in a process of constructing a single chain antibody-toxin fusion protein. Genes encoding a ligand and the toxin are cloned into cDNA using a known cloning method and then they are linked directly or apart by a small peptide iinker. See, for example, Sambrook et al. Molecular Cloning: A Laboratory Manual, Cold Spring Harbor Laboratory (1989).

The present inventors have demonstrated by the incorporation of propidium iodium that this immunotoxin induces cell death (apoptosis) of gene-transfected macrophages, rheumatoid arthritis synovial cells, and FR-β gene-transfected cell lines. The cell to be used for verifying this effect of the immunotoxin can be any cell as long as it is an FR-β expressing cell. Further, the cell death (apoptosis) can also be verified by Annexin-V staining. Further, the action effect can be shown by protein synthesis inhibition due to a decrease in the intracellular incorporation of [³H] uridine or a decrease in the production of cytokines such as TNF-α, IL-1β, IL-6, and IL-8.

### [Base sequences of genes of the H chain and the L chain of FR-β antibody]

The present inventors have amplified the genes of the H chain and the L chain of an FR-β monoclonal antibody (clone 36 or clone 94b) using primers of the Ig-Prime Kit (Novagen). The present inventors incorporate the genes of the H chain and the L chain amplified by the RT-PCR method using Taq polymerase into a pCR(r)2-TOPO(r) vector by the TA cloning method. This vector is transfected into E. coli. The present inventors purify vector inserts from the E. coli and determine their gene sequences using an M13 or T7 primer present in the vector. The vector can be any vector which has T at the 5' end and contains either the M13 or T7 primer.

### [Action effect of FR-β antibody immunotoxin]

The immunotoxin of the present invention is applied to various diseases in which macrophage activation is the major pathological condition and leukemia in which FR-β expressing tumor cells are involved. Since no FR-β expression is observed in macrophages, the action effect is verified using FR-β expressing macrophages with an adenovirus vector.

Further, since no FR-β expression is observed in most cell lines, FR-β expressing cell lines are constructed using a general mammalian expression vector to verify the action effect.

Since macrophages obtained from the rheumatoid arthritis synovial membrane exhibit the FR-β expression, they are suitable to verify the action effect.

### [Dosage and method of administration of FR-β antibody immunotoxin]

Administration is carried out at an effective concentration for the treatment of rheumatoid arthritis, juvenile rheumatoid arthritis, macrophage activation syndrome, septic shock, and acute myeloid leukemia. In order to achieve this purpose, an immunotoxin can be prepared with various excipients which are acceptable and known in this field of technology. Typically, the immunotoxin is administered by injection, intravenously or into a joint cavity. A composition of the present invention is mixed with pharmaceutically acceptable non-oral excipients to formulate into the form of unit dose injections, such as solutions, suspensions, or emulsions. Such excipients are substantially non-toxic and non-therapeutic. Examples of such excipients include physiological saline, Ringer's solution, dextrose solution, and Hank's solution. Non-aqueous excipients such as fixed oil and ethyl oleate can also be used. A preferred excipient is a 5% dextrose in physiological saline solution. Excipients may contain a small amount of additives, for examples, substances to increase isotonicity and chemical stability, including buffer solutions and preservatives.

The amount and the form of administration may vary depending on individuals. Generally, the composition is administered most preferably at a dose of 0.1 to 2 µg/kg as the immunotoxin. Preferably, it is administered by bolus injection. Continuous infusion can also be used. In specific cases, the "therapeutically effective amount" of the immunotoxin of the present invention should be determined as an amount sufficient for the treatment of a patient to cure or at least partly halt a corresponding disease or its complications. The effective amount for such use may vary depending on the severity of the disease and the systemic health condition of the patient. The single administration or muitipie administrations is required depending on the amount and frequency of the administration which are necessary and tolerable to the patient.

Particularly preferred embodiments of the present invention will be described as examples as follows.

### EXAMPLES

### Example 1

Whole RNA (200 µg) was extracted from rheumatoid arthritis synovial cells (1 × 10⁷) with trizole (Gibco BRL) according to the manufacturer's Instruction. An admixture of 5 µl of the whole RNA (1 µg/µl), 1 µl of 10 mM dNTP (dATP, dGTP, dCTP, and dTTP), and 1 µl of oligo (dT) 12-18 primer (0.5 µg/µl) was reacted at 65°C for 5 minutes and then allowed to stand in ice for 1 minute.

Further, 2 µl of 10× RT buffer solution, 24 µl of 25 mM MgCl₂, 2 µl of 0.1 M DTT, and 2 µl of RNase OUTTM were added thereto and the resulting admixture was reacted for 2 minutes. Further, 1 µl of transcriptase (Superscript TM reverse transcriptase, Invitrogen) was added thereto and the resulting admixture was reacted at 70°C for 15 minutes and then allowed to stand in ice for 2 minutes. Further, 1 µl of RNase H was added and the resulting admixture was reacted at 37°C for 20 minutes to complete cDNA synthesis.

After obtaining cDNA, PCR was performed using 4.5 µl of the reaction product, 40 pM each of a sense primer (AGAAAGACATGGTCTGGAAATGGATG) and an antisense primer (GACTGAACTCAGCCAAGGAGCCAGAGTT), 0.6 mM dNTP, and 50 µl of Taq DNA polymerase (1.5 units, Boehringer Mannheim Corp) in 23 cycles of 94°C for 5 minutes, 94°C for 45 seconds, 60°C for 60 seconds, and 72°C for 90 seconds, after which the folate receptor beta (FR-β) gene was amplified by the reaction at 72°C for 10 minutes.

Since the resulting PCR product contains A at the 3' end, it was ligated with a PCR2.1-TOPO vector (Invitrogen) having T at the 5' end. Namely, 1 µl of Solt Solution, 1.5 µl of sterile distilled water, 1 µl of pCR(r)2-TOPO(r) vector were added to 2.5 µl of the PCR product and the resulting admixture was incubated at 22°C for 5 minutes, after which a portion (2 µl) of the incubated admixture was added to one shot E. coli TOP 10F' cells and the resulting admixture was reacted in ice for 30 minutes, after which the reaction solution was treated for heat shock at 42°C for 30 seconds and allowed to stand in ice for 2 to 5 minutes, then 250 µl of S.O.C medium pre-warmed to 37°C was added and the reaction was carried out at 37°C for 1 hour in a shaker. Meantime, an LB plate was warmed to 37°C. The sample added with 40 µl of X-gal (100 mg/ml) and 40 µl of IPTG (20 mg/ml) was admixed into 3.5 ml of LB agar medium and the resulting admixture was poured onto the LB plate and incubated at 37°C overnight.

For cultivation of E. coli cells, a white colony taken from the plate was added to 2 ml of LB medium supplemented with 1 µl of ampicillin (50 mg/ml) and the incubation was carried out in a shaker at 37°C overnight.

DNA purification was carried out using a Qiagen plasmid purification kit (Qiagen). An insert was confirmed by verifying a 783 bp band on an electrophoresis gel after treatment with the EcoRI restriction enzyme. A vector containing the insert was treated with EcoRI and subjected to agarose electrophoresis. The insert part was dissected and subjected to ligation using T4 ligase with the vector pEF-BOS pretreated with EcoRI and alkaline phosphatase (Mizushima et al. pEF-BOS, a powerful mammalian expression vector. Nucleic Acids Res. 1990; 18(17):5322). The ligation product was subjected to transfection into one shot E. coli TOP 10F' cells by the heat shock method. Since the transfected E. coli cells became ampicillin resistant, they were cultured overnight on a 1% agar medium containing ampicillin and the colonies obtained were further cultured overnight in an LB medium supplemented with ampicillin. The resulting E. coli cells were collected and a vector insert was purified by the abovementioned purification method. After treating with the EcoRI restriction enzyme, the insert was confirmed by a 783 bp band on an electrophoresis gel.

Using a mixture of 20 µl of iipofectamine (Gibco BRL), 1 µg of the purified pEF vector insert, and 1 ml of a Hank's balanced salt solution, the FR-β gene was transfected into B300-19 cells which were previously prepared at 1 × 10⁵ in 24 wells. The resulting transfected cells were cultured in a DMEM solution containing G418 (1000 µg/ml) to confirm the FR-β expression of the grown B300-19 cells with an IgM-type anti-FR-β antibody. Namely, 5 × 10⁵ B300-19 cells were reacted with 0.1 ml of the FR-β antibody (1 mg/ml) at 4°C for 30 minutes. The cells were washed 3 times with PBS containing 0.1% NaN₃ and 1% fetal calf serum, after which they were reacted with a fluorescence-labeled goat anti-mouse Ig antibody (BIOSOURCE) at 4°C for 30 minutes. Then, the cells were washed twice with PBS containing 0.1% NaN₃ and 1% fetal calf serum, after which fluorescence of the cells was assayed using EPICS Elite (Coulter). B300-19 cells which consistently express folate receptor-beta (FR-β) were obtained.

### Example 2

A mixture of the FR-β-expressing B300-19 cells (1 × 10⁷) with Freund's complete adjuvant was immunized into 3 places on the back and the abdominal cavity of Balb/C mice. Further, 2 weeks later, a mixture of the B300-19 cells (1 × 10⁷) with Freund's incomplete adjuvant was immunized into the abdominal cavity of Balb/C mice. This immunization was further repeated 2 to 4 times.

Monoclonal antibodies were prepared by the method of Kohler and Milstein (Nature (1975); 256:495-96) or its modified method. The spleen (and several large lymph nodes, if necessary) was dissected and dissociated into single cells. All the dissociated spleen cells were fused with myeloma cells and the hybridomas thus constructed were cultured in a HAT selective medium. Hybridomas which reacted with the immunogen in the culture supernatant were selected.

The hybridomas thus obtained were cultured on plates by the limited dilution method and assayed for production of antibodies which specifically bind to one surface antigen of the immunized cells of interest (not bind to unrelated antigens). Next, selected monoclonal antigen-secreting hybridomas were cultured in vitro (for example, in a tissue culture bottle or using a hollow fiber cell culture system) or in vivo (as a mouse ascites). Further, using the culture supernatant, the isotype and subclass of monoclonal antibodies were determined by a mouse immunoglobulin isotyping ELISA kit (Pharmingen) using anti-mouse immunoglobulin G (IgG) subclass antibodies and anti-mouse isoclass type antibodies.

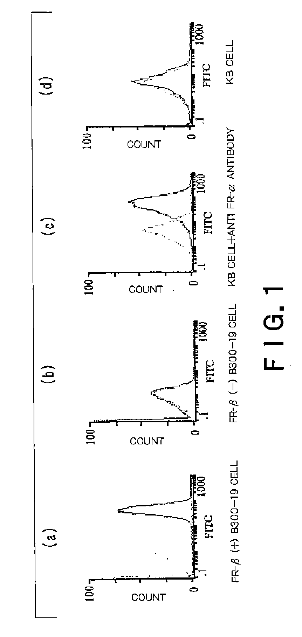

As a result, it was revealed that clone 36 was IgG₂ₐ and clone 94b was IgG₁. The reactivity of antibodies was analyzed by flow cytometry as shown in Example 1. Fig. 1 shows that the obtained clones react with the FR-β gene-transfected cells but not with the KB cells. In analysis by a flow cytometer (see the specification), the X axis shows the number of cells and the Y axis shows the fluorescent intensity of cells. The IgG-type FR-β antibody (clone 36) reacted with the FR-β gene-transfected cells (a) but not with the B300-19 cells which express no FR-β (b). Further, they did not react with the KB cells (c) which express FR-α but not FR-β (d).

### Example 3

The hybridoma cells (1 × 10⁷) were intraperitoneally injected into mice to which 0.5 cc of pristine had been injected 2 weeks earlier into the abdominal cavity and ascites was obtained 2 to 3 weeks later. A 0.5 ml portion of the ascites was loaded onto a protein G column and then the column was washed with a 10-fold volume of phosphate buffer, after which eluate was carried out with 2.5 pH glycine buffer. The pH of the eluate was adjusted to 8.0 with Tris buffer and the eluate was subjected to dialysis with PBS for 24 hours and then concentrated. From 0.5 ml of the ascites, 1 to 2 mg of IgG was obtained.

### Example 4

### [Preparation of Pseudomonas exotoxin from E. coli]

Plasmid pMSS-38-402 for the expression of Pseudomonas exotoxin (PE) ( Onda et al. In vitro and in vivo cytotoxic activities of recombinant immunotoxin 8H9 (Fv)-PE38 against breast cancer, osteosarcoma, and neuroblastoma. Cancer Res. 2004; 64(4):1419-24) and its host E. coli BL21(DE3) (Stratagene) were cultured in 5 ml of LB medium supplemented with 0.1 mg/ml ampicillin and 0.1 mg/ml chloramphenicol at 37°C for 12 to 15 hours. After 12 to 15 hours, 2 L of LB medium was added to 5 ml of the medium and Incubation was continued until the absorbance at a wavelength of 600 nm reached 0.5. When the absorbance at a wavelength of 600 nm reached 0.5, IPTG was added at a concentration of 1 mM to the LB medium and incubation was further continued for 90 minutes.

After completion of the incubation, the cells were recovered and suspended in 50 ml of a 30 mM Tris buffer solution (pH 7.4, containing 20% sucrose and 1 mM EDTA), and the suspension was allowed to stand in ice for 15 minutes. Then, the cells were recovered by centrifugation at 2,000 g for 15 minutes and suspended in 50 ml of sterile distilled water and the suspension was allowed to stand in ice for 15 minutes. Then, centrifugation was carried out at 15,000 g for 15 minutes and the resultant supernatant was collected to obtain a starting material for purification.

### Example 5

Purification of PE was achieved using a Vision Workstation liquid chromatography system (Japan Perceptive). First, the starting material for PE purification was adsorbed at a flow rate of 10 ml/min onto a strong anion exchange resin column (POROS HQ, Poros) which had previously been equilibrated with a 20 mM Tris buffer solution (pH 7.4, containing 1 mM EDTA) and then the column was washed with an excess amount of the same buffer solution. Next, a 20 mM Tris buffer solution (pH 7.4, containing 1 mM EDTA) containing 1 M NaCl was used to set an NaCl concentration gradient from 0% to 100% in 10 minutes. The eluate was fractionated in 2 ml portions from the column at a flow rate of 10 ml/min for PE purification.

The purity of the fractionated sample was confirmed by SDS electrophoresis using the Laemmli method or by assaying for ADP-ribosylation activity. The PE sample after purification was further subjected to molecular size exclusion chromatography (TSK 3000 SW, Toso) with a 100 mM phosphate buffer solution (pH 8.0, containing 0.15 M NaCl and 1 mM EDTA) at a flow rate of 0.35 ml/min to fractionate the eluate in 1 ml portions from the column and thus highly purified PE was obtained.

### Example 6

### [SDS-PAGE]

SDS electrophoresis was carried out according to the Laemmli method (Laemmli-UK, Nature (1970) 227:6680-685). Namely, the plate gel used was a 10% polyacrylamide gel containing 0.1% sodium dodecyl sulfate (SDS) and the running buffer solution was a 25 mM Tris buffer solution containing 130 mM glycine at a final concentration of 0.1%. Each sample solution was prepared with an equal amount of a 100 mM Tris buffer solution (pH 6.5) containing 0.2% SDS and boiled for 5 minutes. After boiling, the sample was loaded on the plate gel and electrophoresis was performed at a constant current of 30 mA. After completion of the electrophoresis, the gel was stained with a 0.05% Coomassie brilliant blue R (Nakarai Tesque) solution and then destained with 10% ethanol containing 7% acetic acid to detect proteins.

### Example 7

### [Assay for ADP-ribosylation activity]

The method of Carroll et al was used (Carroll et al. Active site of Pseudomonas aeruginosa exotoxin A. Glutamic acid 553 is photolabeled by NAD and shows functional homology with glutamic acid 148 of diphtheria toxin. J Biol Chem. 1987; 262(18):8707-11). In the assay for ADP-ribosylation activity, 5 µl of a PE solution (approximately 0.1 to 1.25 µg) was added to 45 µl of 50 mM Tris buffer (pH 8.5, 4 µl of wheat germ extract (Promega), 37 pM ¹⁴CNAD (0.06 µCl), 40 mM DDT, 1 mM EDTA) and the admixture was reacted at 37°C for 10 minutes. After completion of the reaction, 10 µl of trichloroacetic acid (Nakarai Tesque) was admixed and the resultant admixture was centrifuged at 15,000 g for 3 minutes to remove the supernatant. The precipitate was further washed by the addition of a 5% trichloroacetic acid solution and centrifugation. After the washing, the ¹⁴C radioactivity of the precipitate was measured using a liquid scintillation counter to obtain an index of the ADP-ribosylation activity.

### Example 8

### [Construction of immunotoxin]

The method of Haasan et al was generally used (Haasan et al. Anti-tumor activity of K1-LysPE38QQR, an immunotoxin targeting mesothelin, a cell-surface antigen overexpressed in ovarian cancer and malignant mesothelioma. J Immunother. 2000 J; 23(4):473-9).

The coupling of an IgG monoclonal antibody against a human FR-β antigen (clone 36) with succinimidyl trans-4-(maleimidylmethyl)cyclohexane-1-carboxylate (SMCC, Sigma-Aldrich) was carried out. Namely, 100 µg of SMCC was added to 1 ml of a clone 36 antibody solution which was prepared at a protein concentration of 3.0 mg/ml using a 100 mM phosphate buffer solution and the admixture was reacted at room temperature for 1 hour.

After completion of the reaction, excess SMCC was removed using a desalting chromatography column PD-10 (Amersham Pharmacia) and a 100 mM phosphate buffer solution (pH 6.5, containing 150 mM NaCl and 1 mM EDTA). The efficiency of the coupling of the clone 36 antibody with SMCC was determined by measuring absorbance at a wavelength of 412 nm using a DTNB (dithiobis, Sigma-Aldrich) reagent and converting the measurement using the molecular extinction coefficient of DPNB per mole, 13,600. As a result, 2.8 to 3.1 molecules of SMCC were coupled with one molecule of the clone 36 antibody.

Next, the coupling of PE and succinimidyl 3-(2-pyridyldithio)propionate (SPDP, Sigma-Aldrich) was carried out. Namely, 400 µg of SPDP was added to 1 ml of a PE solution which was prepared at a protein concentration of 10 mg/ml using a 100 mM phosphate buffer solution (pH 6.5, containing 150 mM NaCl and 1 mM EDTA) and the admixture was reacted at 4°C for 12 to 15 hours.

After completion of the reaction, excess SPDP was removed using a desalting chromatography column PD-10 (Amersham Pharmacia) and a 100 mM phosphate buffer solution (pH 6.5, containing 0.15 M NaCl and 1 mM EDTA). The efficiency of the coupling of PE and SPDP was determined by measuring absorbance at a wavelength of 343 nm using 2-mercaptoethanol (sigma-Aldrich) and converting the measurement using the molecular extinction coefficient of SPDP per mole, 8,080. As a result, 1.2 to 1.5 molecules of SPDP were coupled with one molecule of PE.

The coupling of the clone 36 antibody-SMCC with PE-SPDP was carried out using 3 mg of the clone 36-SMCC and 6 mg of the PE-SPDP.

First, 100 µg of tris-2-carboxyethylphosphine (TCEP, Molecular Probes) was added to 6 mg equivalent of the PE-SPDP (in a 100 mM phosphate buffer solution (pH 6.5) containing 150 mM NaCl and 1 mM EDTA) and the admixture was reacted at room temperature for 20 minutes to activate the PE-SPDP.

After completion of the reaction, 3 mg equivalent of the clone 36 antibody (in a 100 mM phosphate buffer solution (pH 6.5) containing 150 mM NaCl and 1 mM EDTA) was admixed in a centrifuge concentrator (Centricon 10, Amicon) with a molecular weight cut-off of 10,000 and centrifuged at 4,800 g at 4°C to make a final protein concentration of 5-7 mg/ml. After the centrifugation, the resulting protein solution was reacted at 4°C for 15 to 18 hours.

After completion of the reaction, substitution of the protein solution was carried out using a desalting chromatography column PD-10 (Amersham Pharmacia) and a 20 mM Tris buffer solution (pH 7.4, containing 1 mM EDTA) to prepare a starting material for immunotoxin purification.

Purification of immunotoxin was carried out according to the abovementioned method for PE purification. First, the starting material for immunotoxin purification was adsorbed at a flow rate of 10 ml/min onto a strong anion exchange resin column (POROS HQ, Poros) which had been previously equilibrated with a 20 mM Tris buffer solution (pH 7.4, containing 1 mM EDTA) and then the column was washed with an excess amount of the same buffer solution. Next, a 20 mM Tris buffer solution (pH 7.4, containing 1 mM EDTA) containing 1 M NaCl was used to set an NaCl concentration gradient from 0% to 100% in 10 minutes. The eluate was fractionated from the column in 2 ml portions at a flow rate of 10 ml/min for immunotoxin purification.

The purity of the fractionated sample was confirmed using the abovementioned SDS electrophoresis by the Laemmli method and by assaying for ADP-ribosylation activity. The immunotoxin after purification was further subjected to molecular size exclusion chromatography (TSK 3000 SW, Toso) using a 50 mM phosphate buffer solution (pH 7.3, containing 150 mM NaCl) to obtain a highly purified immunotoxin. The highly purified immunotoxin was further treated with a sterilization filter and stored at -80°C (at a final concentration of 0.1 to 0.2 mg/ml).

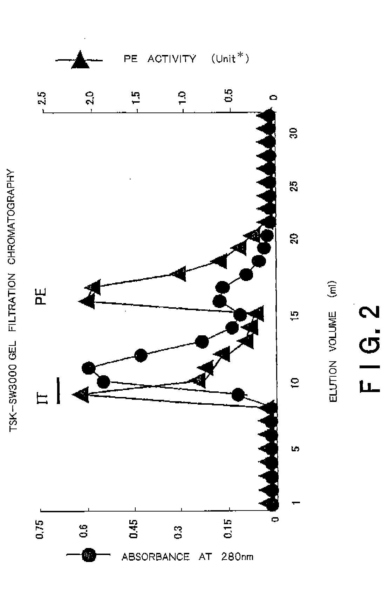

Fig. 2 shows the result of gel filtration chromatography of the anti-FR-β antibody immunotoxin using TSK-SW3000. The X axis shows the elution volume and the Y axis shows the protein concentration at OD 280 with the solid circle and the ADP-ribosylation activity of Pseudomonas exotoxin with the solid triangle. The first peak of the protein concentration has a higher molecular weight and is considered to be the antibody or the antibody conjugated with the toxin. The next peak has a smaller molecular weight and is considered to be the toxin. Both peaks showed the ADP-ribosylation activity.

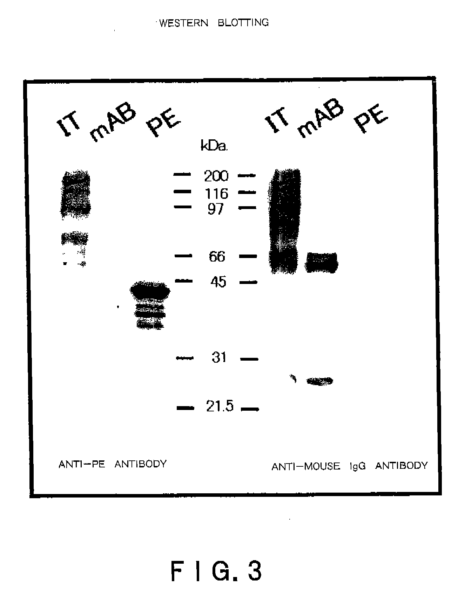

Fig. 3 is the result of Western blotting in which the FR-β antibody immunotoxin (IT) conjugate, the FR-β antibody (mAB), and Pseudomonas exotoxin (PE) were electrophoresed using SDS-PAGE and subjected to Western blotting with an anti-PE antibody and an anti-mouse IgG antibody. Only IT showed bands reacting both antibodies from 66 kDa to 200 kDa.

### Example 9

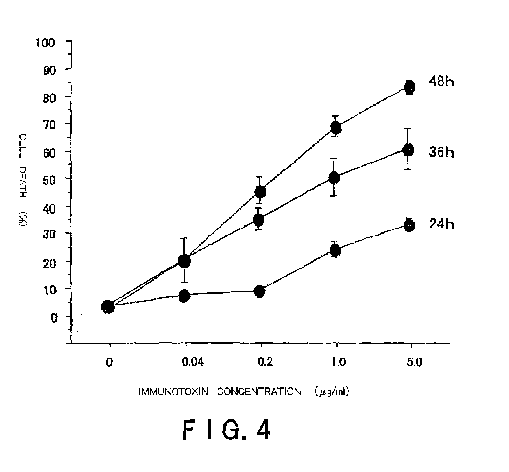

Cells used were B300-19 cells in which FR-β was consistently expressed in Example 1. Toxicity of the immunotoxin was measured by the binding of propidium iodide and DNA using a flow cytometer (Nicolletti et al. A rapid and simple method for measuring thymocyte apoptosis by propidium iodide staining and flow cytometry. J Immunol Methods. 1991; 139(2):271-9). Specifically, the B300-19 cells (2 × 10⁵/ml) and the FR-β antibody immunotoxin at various concentrations were incubated for various times. The resulting B300-19 cells were washed once with PBS, 0.5 ml of propidium iodide (40 µg/ml) was added to the cell pellet obtained and the admixture was reacted at room temperature overnight, after which the fluorescence of the cells was measured by a flow cytometer. Cells which were stained poorly with propidium iodide were considered to be dead cells and the fluorescence was measured using a flow cytometer. The result of the measurement is shown in Fig. 4.

Fig. 4 shows the rate of cell death (shown in the Y axis) 24 hours, 36 hours, and 48 hours after mixing the B300-29 cells and the FR-β antibody immunotoxin in various concentrations (shown in the X axis). In Fig. 4, data are the average of four experiments and error bars indicate SDs.

### Example 10

cDNA of the FR-β was incorporated into a pEF-BOS vector, E. coli was transfected with the resulting vector by the heat shock method and then cell colonies were grown overnight to select an ampicillin-resistant insert positive clone. The positive clone was grown on 2 ml of LB medium and cDNA was purified using a Qiagen plasmid purification kit (Qiagen).

The FR-β gene was isolated from the plasmid by treating with the restriction enzyme XbaI and after ethanol precipitation, the FR-β gene was blunted using a DNA Blunting Kit (Takara), after which the resulting gene was extracted from the gel using QIAEXII (Takara) after electrophoresis. After phenol/chloroform extraction, ethanol precipitation was carried out and the resulting precipitate was dissolved in water.

The insert and a cosmid vector pAxCAwt were ligated and subjected to ethanol precipitation. The resulting mixture was cleaved with SwaI. The resulting fragments were transfected into E. coli DH5α using a Gigapack 3 Gold Packing Extract (Stratagene). The resulting E. coli cells were plated on an agar plate containing ampicillin and the grown colonies were picked up and cultured in 10 ml of an LB medium supplemented with ampicillin, after which plasmids were recovered by the alkaline solution method and subjected to the PEG precipitation.

The precipitate was dissolved in water and the direction and the structure of the Insert were confirmed by electrophoresis using restriction enzymes XbaI and BamHI. Cosmids having forward and backward inserts were cultured in 2 I of LB medium supplemented with ampicillin for large scale purification using a Large Construction Kit (Qiagen).

According to an Adenovirus Expression Vector Kit (Takara), the product was subjected to cotransfection with DNA-TP, which had been treated with restriction enzymes, by the calcium phosphate method using a CellPhect Transfection Kit (Amersham Pharmacia Biotech). Briefly, 9 µl of pAxCAwt (8.1 µg) and 10 µl of DNA-TPC (7 µg) were mixed with 101 l of distilled water and the mixture was transfected by the calcium phosphate method into L293 cells grown in a 3.5 ml Falcon dish at a confluence of 80%.

After 24 hours, undiluted, 10-fold diluted and 100-fold diluted suspensions of the resulting L293 cells were prepared, transferred into a 96-well plate and then cultured for about 20 days. Recombinant adenovirus in which intracellular recombination occurred was obtained in a dead cell culture supernatant. Recombinant cosmids in 10-fold dilution and 100-fold dilution wells were confirmed by treating the cells with % SDS and then with phenol/chloroform and cleaving the cosmids with restriction enzymes XbaI and BamHI to individually confirm the presence of 768 bp and 1703 bp inserts using 1% agar gel.

The culture supernatant in which the inserts were confirmed was frozen and thawed 5 times and centrifuged at 3000 rpm for 10 minutes to obtain the supernatant. The supernatant was added to a 200 ml flask in which L293 cells were grown at a confluence of 80% and after 4 days, the supernatant was obtained from dead cell wells. The same procedure was repeated to obtain a virus with a high titer.

The titer of the virus was determined using the 50% tissue culture infectious dose method (Precious B and Russel W.C. (1985) in Virology: A Practical Approach. ed. Mahy B.W.J. (IRL Oxford), pp. 193-205).

### Example 11

A blood sample was taken from the vein of a healthy individual using a 50 ml heparin-containing syringe (200) and diluted 3-fold with PBS. The diluted blood was layered over a 50 ml tube containing 15 ml of Ficoll-Hypaque and centrifuged at room temperature at 3000 g for 15 minutes to isolate nucleated cells.

The upper layer was collected, PBS was added, and the admixture was centrifuged at 2000 g for 5 minutes, after which the supernatant was discarded, the pellet was loosened, PBS was added, and the admixture was centrifuged at 1000 g for 5 minutes. The pellet was loosened and cultured at a cell concentration of 1 × 10⁶/ml in a Falcon dish for 30 minutes and after washing 10 times with PBS, adhered cells were scraped off using a rubber policeman. After centrifugation, the cells were prepared at 1 × 10⁶/ml in DMEM and adhered again in a dish to obtain adhered cells. The adhered cells were cultured for 24 hours using M-CSF and then transfected at a MOI of 100 with an adenovirus vector carrying the folate receptor beta gene or an adenovirus vector carrying the reverse folate receptor beta gene.

The transfection experiment was carried out by adding the virus supernatant and centrifuging at 37°C at 3000 g for 1 hour. The cells were prepared at 5 × 10⁶/ml and cultured for 72 hours. Further, to the transfected cells, an FR-β antibody and an FITC-labeled anti-mouse immunoglobulin antibody were added in sequence and positive cells were measured by a flow cytometer.

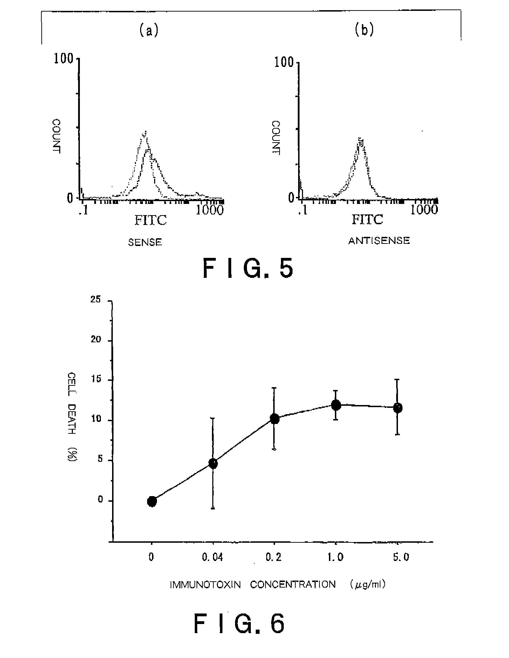

Fig. 5 shows the FR-β expression of macrophages by the introduction of the sense FR-β adenovector.

In Fig. 5(a), the sense FR-β gene was introduced and the reaction was carried out with the FR-β antibody and the FITC-labeled anti-mouse Ig antibody. In Fig. 5(b), the antisense FR-β gene was introduced and the reaction was carried out with the FR-β antibody and the FITC-labeled anti-mouse Ig antibody. Fluorescence was measured by flow cytometry. The X axis represents fluorescence and the Y axis represents the number of cells.

### Example 12

Macrophages adjusted to 1 × 10⁶/ml were cultured in a 24-well dish at 1 ml per well, the measurements of the concentration of the FR-β antibody immunotoxin and cell death were carried out in the same manner as in Example 6. Fig. 6 shows cell death of the FR-β expressing macrophages by the FR-β antibody immunotoxin.

The macrophages in which the sense FR-β gene was introduced were mixed with the FR-β antibody immunotoxin in various concentrations (shown in the X axis) and the rate of cell death was obtained after 72 hours while the macrophages in which the antisense FR-β gene was introduced were mixed with the FR-β antibody immunotoxin in various concentrations and the rate of cell death was obtained after 72 hours. In Fig. 6, the difference of the two rates was shown in the Y axis. The cells poorly stained with propidium iodide were considered to be dead cells and fluorescence was measured using a flow cytometer. In Fig. 6, the data are the averages obtained in the experiment using the macrophages from four healthy individuals and the error bars indicate SDs.

### Example 13

Synovial cells were purified from the synovial membrane obtained from a rheumatoid arthritis patient upon knee joint replacement surgery. First, the synovial membrane was cut into about 5 mm pieces and treated with 30 ml of DMEM containing 1 mg/ml collagenase type 5 at 37°C for 30 minutes. After removing debris with a stainless mesh, an equal volume of DMEM was added and the admixture was centrifuged at room temperature at 2000 g for 15 minutes using the Ficoll-Hypaque density gradient centrifuge method, after which the upper layers were collected, a 2-fold volume of DMEM was added, and the admixture was centrifuged at 1500 g, 1000 g to obtain synovial nucleated cells.

The cells (1 × 10⁷) obtained were added to a Falcon dish, cultured at 37°C for 30 minutes, and then washed 10 times with PBS to obtain adhered cells. The adhered cells were scraped off from the dish using a rubber policeman and collected into a 50 ml tube. After repeating the cell adhesion described above, the 50 ml tube was centrifuged at 1000 g. The cells prepared at a concentration of 1 × 10⁶ cells/ml were cultured in DMEM supplemented with 10% human serum and 10% fetal calf serum. A mixture of equal amounts of an antibody and a toxin was used as a control added with immunotoxin. Apoptosis of the cells after 72 hours was measured by the method described above.

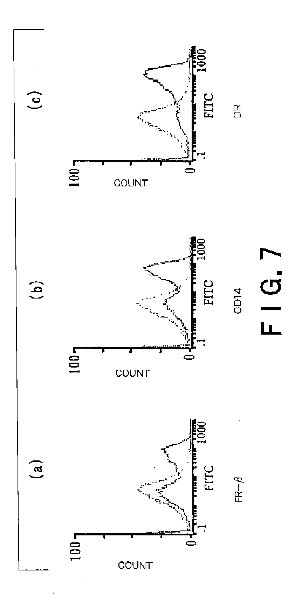

Fig. 7 shows that the rheumatoid arthritis synovial cells express FR-β. The rheumatoid arthritis synovial cells were reacted with the FR-β antibody (a), CD14 antibody (b), and DR antibody, after which they were reacted with the FITC-labeled anti-mouse Ig antibody. Fluorescence was measured using a flow cytometer. The X axis represents the number of cells and the Y axis represents fluorescence. The expression of FR-β was observed more than 40% of the rheumatoid arthritis synovial cells.

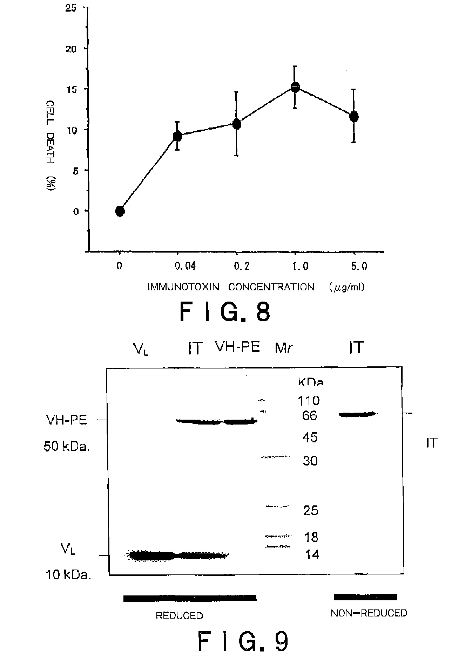

Fig. 8 shows cell death of the rheumatoid arthritis synovial cells by the FR-β antibody immunotoxin. The rheumatoid arthritis synovial cells were mixed with the FR-β antibody immunotoxin in various concentrations (shown in the X axis) and the rate of cell death was obtained after 72 hours while the rheumatoid arthritis synovial cells were mixed with a mixture of equal amounts of molecules of the FR-β antibody and the toxin and the rate of cell death was obtained after 72 hours. In Fig. 8, the difference of the two rates is shown in the Y axis. The cells poorly stained with propidium iodide were considered to be dead cells and fluorescence was measured using a flow cytometer. In Fig. 8, the data are the averages experimentally obtained from six rheumatoid arthritis cases and the error bars indicate SDs.

### Example 14

To 5-10 × 10⁶ hybridoma cells was added 0.75 ml of a TRIZOL(r) LS reagent solution and the admixture was allowed to stand at 15 to 30°C for 5 minutes. Further, 0.2 ml of chloroform per 0.75 ml of the TRIZOL LS reagent solution was added and the admixture was stirred and then allowed to stand at 15 to 30°C for 2 to 15 minutes. After centrifugation at 12000 g at 4°C for 15 minutes, only the top transparent layer was transferred to a separate tube.

To the solution thus obtained was added 0.5 ml of isopropyl alcohol per 0.75 ml of the TRIZOL(r) LS reagent solution, and the admixture was allowed to stand at 15 to 30°C for 10 minutes. After centrifugation at 12000 g at 4°C for 10 minutes, the supernatant was discarded, 1 ml of 75% ethanol per 0.75 ml of the TRIZOL(r) LS reagent solution was added, and after centrifugation at 7500 g at 4°C for 5 minutes, the supernatant was discarded. This procedure was repeated and then the resulting sample was dried. Before drying was complete, 10 µl of sterile distilled water without DNase and RNase was added. Sterile distilled water without DNase and RNase was added to make a total RNA concentration of 1 µg/µl. To a 5 µl portion of the admixture were added 1 µl of 10 mM dNTP mix and 1 µl of an oligo(dT) 12-18 primer (0.5 µg/µl), and the resulting admixture was incubated at 65°C for 5 minutes and then allowed to stand in ice for 1 minutes. Further, 2 µl of a 10× RT buffer solution, 4 µl of 25 mM MgCl₂, 2 µl of 0.1 M DTT, and 2 µl of RNase OUTTM were added and the admixture was incubated at 42°C for 2 minutes; µl of transcriptase (SuperScript TM2RT) was added and the admixture was incubated at 70°C for 15 minutes and then allowed to stand in ice for 2 minutes; and finally 1 µl of RNase H was added and the admixture was incubated at 37°C for 20 minutes to obtain cDNA.

The cDNA (1 µl each) was added into 13 reaction tubes, each containing the Ig-Prime Kit (Novagen), 1 unit of Taq DNA polymerase, 50 µM each of dATP, dCTP, dGTP, and dTTP, 40 mM Tris-hydrochloric acid (pH 9.0), and 21.5 mM MgCl₂, and into each tube, 0.5 µl of the 5' primer and 0.5 µl of the 3' primer for the H chain and the L chain genes were added.

PCR was performed with 27 cycles each consisting of 94°C for 1 minute, 50°C for 1 minute, and 72°C for 2 minutes, and a final extension step of 72°C for 6 minutes. For the determination of the base sequences of clone 36 and clone 94b, the 5' primer MuIgVH5'-B and the 3' primer MuIgVH3'-2 were used for the H chain genes and the 5' primer MuIgκVL5'-A and the 3' primer MuIgκVL3'-1 were used for the L chain genes. To 2.5 µl each of the PCR products were added Salt Solution (0.5 unit of T4 DNA ligase), 1 µl of sterile distilled water, 1.5 µl of, and 1 µl of pCR(r) 2-TOPO(r) vector, and the admixture was incubated at 22°C for 5 minutes. A 2 µl portion of the incubated admixture was added to one shot E. coli (TOP 10F') cells and kept in ice for 30 minutes, treated for heat shock at 42°C for 30 seconds, and kept in ice for 2 to 5 minutes, after which 250 µl of an S.O.C medium pre-warmed to 37°C was added and the incubation was carried out in a shaker at 37°C for 1 hour. Meantime, an LB plate was warmed to 37°C and a mixture of the sample with 40 µl of X-gal (100 mg/ml) and 40 µl of IPTG (20 mg/ml) was mixed with 3.5 ml of LB agar medium and the resulting admixture was poured onto the LB plate and incubated at 37°C overnight.

A white colony taken from the plate was added into 2 ml of LB medium supplemented with 1 µl of ampicillin (50 mg/ml) and the incubation was carried out at 37°C overnight. DNA purification was performed using a Qiagen plasmid purification kit (Qiagen).

Base sequences were determined using a Big Dye Terminator V3.1 Cycle Sequencing Kit (Applied Biosystems). Namely, to a 5.3 µl portion of the purified DNA solution (25 µl) were added 4 µl of a Ready Reaction Mix and then further 0.7 µl of an M13R primer or a T7 primer.

PCR was performed with 25 cycles each consisting of 96°C for 10 seconds, 50°C for 5 seconds, and 60°C for 4 minutes, and then the reaction solution was allowed to stand at 4°C. To 10 µl of the PCR product were added 1 µl of 3 M sodium acetate and 10 µl of 100% ethanol and the admixture was allowed to stand at -20°C for 20 minutes and then centrifuged at 15000 g at 4°C for 10 minutes, after which the supernatant was discarded, 180 µl of 70% ethanol was added and the admixture was stirred and centrifuged at 15000 g at 4°C for 5 minutes, after which the supernatant was discarded and DNA was dried. To the DNA was added 15 µl of a template suppression reagent solution, and the admixture was stirred, subjected to centrifuge flash, stirring and further centrifuge flash, incubated at 99°C for 5 minutes, then placed in ice and subjected to base sequence analysis using an ABI310 sequencer.

### Example 15

### [Introduction of cysteine mutation in the variable region of immunoglobulin heavy chain]

A mutation was introduced into the plasmid pCR2.1-TOPO/94bVH containing the VH gene of clone 94b obtained in Example 14, using a Quick Change Site-Directed Mutagenesis Kit (Stratagene) with primers (cagaggcctgaacagtgtctggagtggattggaag and cttccaatccactccagacactgttcaggcctctg) which were designed to cause mutation of the amino acid glycine (base sequence ggc) at position 63 of the immunoglobulin clone 94b heavy chain variable region (VH) into cysteine (base sequence tgt).

This PCR reaction was carried out with 12 cycles consisting of 95°C for 30 seconds, 55°C for 1 minute and 68°C for 4 minutes, after treating the reaction solution at 95°C for 30 seconds.

Next, the DNA after the reaction was transfected into E. coli (XL1-Blue supercompetent cell) and a transformant was selected using LB medium containing 100 µl/ml of ampicillin. The plasmid of the selected transformant was extracted using a DNA purification kit (QIAprep Spin Miniprep Kit, Qiagen). Further, its base sequence was determined by an ABI310 sequencer using an M13 reverse primer (caggaaacagctatgac) and a base sequencing kit (Big Dye Terminator V3.1 Cycle Sequencing Kit, Applied Biosystems) to confirm that glycine at position 63 (base sequence ggc) was mutated to cysteine (base sequence tgt).

### Example 16

### [Insertion of the immunoglobulin heavy chain variable region gene with the introduced mutation into pRK79/PE38 vector]

Next, the clone 96b VH gene with the introduced mutation was inserted into a pRK79 vector having the PE38 gene (PRK79/PE38) as follows.

As annealing primers for the 5' end (FR1) and the 3' end (JK) of the clone 94b VH gene with the introduced mutation, taagaaggagatatacatatggaggttcagctgcagcagtc and gccctcgggacctccggaagcttttgaggagactgtgagagtgg were designed, respectively. The FR1 annealing primer contains a restriction enzyme NdeI site and protein expression is possible by cloning at this site using atg in the site as a start codon. The JK annealing primer is designed to place "a" next to the JK annealing sequence followed by a restriction enzyme Hind III site so that the clone VH gene and the PE38 gene on the vector pRK79 can be ligated in the same frame by cloning at the restriction enzyme Hind III site.

Using the combination of these primers and DNA polymerase (Pfu DNA polymerase, Stratagene), PCR was performed with the pCR2.1-TOPO/94bVH plasmid into which the mutation was introduced.

This PCR reaction was carried out after 1 cycle of 95°C for 4 minutes, with 30 cycles consisting of 95°C for 1 minute, 54°C for 1 minute, and 72°C for 1 minute, followed by 1 cycle of 72°C for 10 minutes.

Next, the PCR product was subjected to electrophoresis and DNA having a size of interest was recovered from the gel using a QIAquick Gel Extraction Kit (Qiagen). Further, the recovered PCR product was cleaved with restriction enzymes Hind III (New England Biolabs) and NdeI (New England Biolabs). The VH gene with the introduced mutation treated with the restriction enzymes was mixed with the pRK79/PE38 treated with the same restriction enzymes and the admixture was subjected to a ligation reaction at 16°C overnight using a Ligation High kit (Toyobo).

Next, the ligation product was transfected into E. coli (TOP 10F', Invitrogen) and a transformant was selected using LB medium supplemented with 100 µg/ml ampicillin.

The DNA of the transformant was extracted using a DNA purification kit (QIAprep Spin Miniprep Kit, Qiagen) and the base sequence of the plasmid was determined by an ABI310 sequencer using a T7 promoter primer (taatacgactcactataggg) and a base sequencing kit (Big Dye Terminator V3.1 Cycle Sequencing Kit, Applied Biosystems) to confirm that the VH gene with the introduced mutation was ligated to the PE38 base sequence in the T7 promoter downstream region on the pRK79 vector.

### Example 17

### [Introduction of cysteine mutation into the immunoglobulin light chain variable region]

The amino acid glycine at position 125 of the immunoglobulin clone 94b light chain variable region (VL) was mutated to cysteine and the VL gene with the Introduced mutation was inserted into the pRK79 vector as follows. As a 5' end annealing primer, taagaaggagatatacatatggacattgtgatgtcacaatc was designed. Since this primer contains a restriction enzyme NdeI site, protein expression is possible by cloning at this site using atg as a start codon.

As a 3' end (JK) annealing primer, gctttgttagcagccgaattcctatttgatttccagcttggtgccacaaccgaacgt was designed. This primer was designed to mutate the glycine (gga) at position 125 into cysteine (tgt) and place a stop codon tag followed by a restriction enzyme EcoRI site. Using the combination of these primers and DNA polymerase (Pfu DNA polymerase, Stratagene), PCR was performed with the plasmid pCR2.1-TOPO/94bVL containing the clone 94b VL gene obtained in Example 14.

This PCR reaction was carried out after 1 cycle of 95°C for 4 minutes, with 30 cycles consisting of 95°C for 1 minute, 54°C for 1 minute, and 72°C for 1 minute, followed by 1 cycle of 72°C for 10 minutes.

### Example 18

### [Insertion of the immunoglobulin light chain variable region gene with the introduced mutation into pRK79 vector]

The PCR product was subjected to electrophoresis and DNA having a size of interest was recovered from the gel using a DNA purification kit (QIAquick Gel Extraction Kit, Qiagen).

The recovered PCR product was cleaved with the restriction enzyme EcoRI (New England Biolabs) and the restriction enzyme NdeI (New England Biolabs) and then mixed with the pRK79 plasmid cleaved with the same enzymes and the mixture was subjected to a ligation reaction at 16°C overnight using a Ligation High kit (Toyobo). Next, the ligation product was transfected into E. coli TOP 10F' (Invitrogen) and a transformant was selected using LB medium supplemented with 100 µg/ml ampicillin.

A plasmid was extracted from the transformant using a DNA purification kit (QIAprep Spin Miniprep Kit, Qiagen) and its base sequence was determined by an ABI310 sequencer using a T7 promoter primer (taatacgactcactataggg) and a base sequencing kit (Big Dye Terminator V3.1 Cycle Sequencing Kit, Applied Biosystems) to confirm that the glycine (gga) at position 125 of the VL with the introduced mutation was mutated into cysteine (tgt), that the ligation was to the T7 promoter downstream region on the pRK79 vector, and that the stop codon tag was located next to the JK sequence.

### Example 19

### [Preparation of recombinant protein inclusion body]

E. coli BL21(DE3λ) was transfected using 50 ng of the plasmid pRK79/PE38 in which the abovementioned VH gene with the introduced mutation was incorporated or the plasmid pRK79 in which the VL gene with the introduced mutation was incorporated.

Selection of the E. coli in which the gene was transfected was carried out by incubation at 37°C for 15 to 18 hours in an LB medium supplemented with ampicillin (100 µg/ml) and chloramphenicol (20 µg/ml).

E. coli cells after completion of the incubation for selection were cultured in 500 ml of a Super Broth medium supplemented with ampicillin (100 µg/ml) and chloramphenicol (20 µg/ml) at 37°C until the absorbance at a wavelength of 600 nm reached 0.6.

Further, 1 mM IPTG (isopropyl-beta-D-thio-galactopyranoside) was added and incubation was carried out at 37°C for 90 minutes. E. coli cells after completion of the incubation were recovered by centrifugation and then suspended using a 50 mM Tris buffer solution (pH 7.4, containing 20 mM EDTA) and the suspension was made a final volume of 20 ml with the same buffer solution and transferred into a homogenizer. Egg white lysozyme was added to 20 ml of the suspension transferred into the homogenizer at a final concentration of 0.2 mg/ml and the admixture was reacted at room temperature for 1 hour to decompose the E. coli cell component. After decomposition, 2.5 ml each of a 5M NaCl solution and a 25% Triton-X solution were added and the admixture was homogenized and then allowed to react at room temperature for 60 minutes. After completion of the reaction, the precipitate was recovered by centrifugation at 20,000× g at 4°C.

The recovered precipitate was resuspended in 20 ml of the same Tris buffer, 2.5 ml each of a 5M NaCl solution and a 25% Triton-X solution were added and the admixture was homogenized and centrifuged at 20,000× g at 4°C to recover the precipitate. After repeating this procedure 8 times, the precipitate was resuspended in 20 ml of the same Tris buffer solution and the suspension was homogenized and then centrifuged at 20,000x g at 4°C to recover the precipitate. This procedure was repeated 5 times and the resultant precipitate to be used as a recombinant immunotoxin inclusion body was further dissolved in a 0.1 M Tris buffer solution (pH 8.0, containing 10 mM EDTA and 6 M guanidine hydrochloride) to make a final concentration of 10 mg/ml with the same buffer solution and stored at -80°C.

### Example 20

### [Construction of recombinant double chain Fv anti-FR-β PE antibody]

The recombinant protein inclusion body solution stored at -80°C was thawed at room temperature and 0.5 ml of VH and 0.25 ml of VL were individually transferred into a 1.5 ml tube. Next, dithiothreitol (DTT) was added at a final concentration of 10 mg/ml to carry out reducing treatment at room temperature for 4 hours. After the reducing treatment, 0.5 ml of VH and 0.25 ml of VL were mixed and dissolved in 75 ml of a 0.1 M Tris buffer solution (pH 8.0, containing 0.5 M arginine, 0.9 mM oxidized glutathion, and 2 mM EDTA). This solution was allowed to stand at 10°C for 40 hours to ligate VH and VL. After completion of the ligation, the solution was concentrated to a volume of 5 ml using a centrifuge concentrator (Centricon 10, Amicon) with a cut-off molecular weight of 10,000 and further diluted with 50 ml of distilled water. This diluted solution was used as a starting material for recombinant immunotoxin purification.

### Example 21

### [Purification of recombinant double chain Fv anti-FR-β PE antibody]

First, the abovementioned starting material for purification was adsorbed onto a strong anion-exchange resin column (Hi-trap Q, Amersham Pharmacia) previously equilibrated with a 20 ml Tris buffer solution (pH 7.4, containing 1 mM EDTA) at a flow rate of 30 ml/hour and the column was washed with a 20 mM Tris buffer solution (pH 7.4, containing 1 mM EDTA) until the absorbance at 280 nm reached less than 0.005. Next, elution was carried out with a 20 mM Tris buffer solution (pH 7.4, containing 1 mM EDTA) containing 0.3 M NaCl. After the elution, the eluate was subjected to dialysis/desalting in a 20 mM Tris buffer solution (pH 7.4, containing 1 mM EDTA).

Next, using a perfusion chromatography system (Applied Biosystems) and a strong anion-exchange column (POROS HQ, Poros), further purification was carried out. The dialyzed material for purification was adsorbed onto the column previously equilibrated with a 20 mM Tris buffer solution (pH 7.4, containing 1 mM EDTA) at a flow rate of 10 ml/min. After the adsorption, the column was washed with the same buffer solution, and then the purification of recombinant immunotoxin was carried out using a NaCl concentration gradient (setting the concentration to reach from 0 M to 1 M in 10 minutes). The eluate from the column was fractionated in 2 ml portions and a fraction with a high degree of purity was considered as a purified recombinant immunotoxin. The degree of purity was confirmed by the Laemmli method using SDS electrophoresis as described below.

### Example 22

### [Removal of endotoxin]

Endotoxin in the purified recombinant immunotoxin was removed using a perfusion chromatography system (Applied Biosystems) and size exclusion chromatography (TSK 3000 SW, Toso). First, the chromatography system and the size exclusion chromatography column were washed with 75% ethanol for disinfection for 48 hours and then further washed with distilled water for injection (Japanese Pharmacopoeia, Otsuka Pharmaceutical Co.). After washing with distilled water, the size exclusion chromatography column was equilibrated with physiological saline (Japanese Pharmacopoeia, Otsuka Pharmaceutical Co.). After completion of the equilibration, the recombinant immunotoxin after purification was loaded onto the size exclusion chromatography column and then the eluate from the column was fractionated at a flow rate of 0.25 ml/min. The highly purified recombinant immunotoxin after purification was further treated with a sterilizing filter, a portion of the filtered fraction was used to measure the protein concentration and the rest was stored at -80°C. In this way, 0.15 mg of a recombinant immunotoxin with a high degree of purity without endotoxin was obtained from 7.5 mg of the recombinant immunotoxin inclusion body.

### Example 23

### [SDS-PAGE]

SDS electrophoresis was carried out according to the Laemmli method. Namely, the plate gel used was a 10% polyacrylamide gel containing 0.1% sodium dodecyl sulfate (SDS) and the running buffer solution was a 25 mM Tris buffer solution containing 130 mM glycine at a final concentration of 0.1%. Each sample was prepared with an equal amount of a 100 mM Tris buffer solution (pH 6.5) containing 0.2% SDS and boiled for 5 minutes. After boiling, the sample was loaded on the plate gel and electrophoresis was performed at a constant current of 30 mA. After completion of the electrophoresis, the gel was stained with a 0.05% Coomassie brilliant blue R solution and then destained with 10% ethanol containing 7% acetic acid to detect proteins.

Fig. 9 shows the SDS-polyacrylamide electrophoresis pattern of the recombinant double chain Fv anti-FR-β PE antibody. The recombinant double chain Fv anti-FR-β PE chimeric antibody (molecular weight: 60 kDa) was decomposed into V_{H}-PE (50 kDa) and V_{L} (10 kDa) by reduction. Each lane from left to right shows VL protein, recombinant double chain Fv anti-FR-β PE antibody (IT), VH-PE fusion protein electrophoresed under reducing conditions, molecular weight markers (Mr), and recombinant double chain Fv anti-FR-β PE antibody (IT) under non-reducing conditions.

### Example 24

### [Construction of FR-β expressing HL-60 cells]

The PCR2.1-TOPO/FR-β obtained in Example 4 was treated with the restriction enzyme EcoRI and mixed with a vector pCDNA3 (Invitrogen) treated with the same restriction enzyme and the mixture was subjected to a ligation reaction. Gene transfection into human acute myeloid leukemia cell line HL-60 cells was carried out in the same manner as described in Example 1 to obtain an FR-β expressing HL-60 cell line.

### Example 25

### [Action of recombinant double chain Fv anti-FR-β PE antibody]

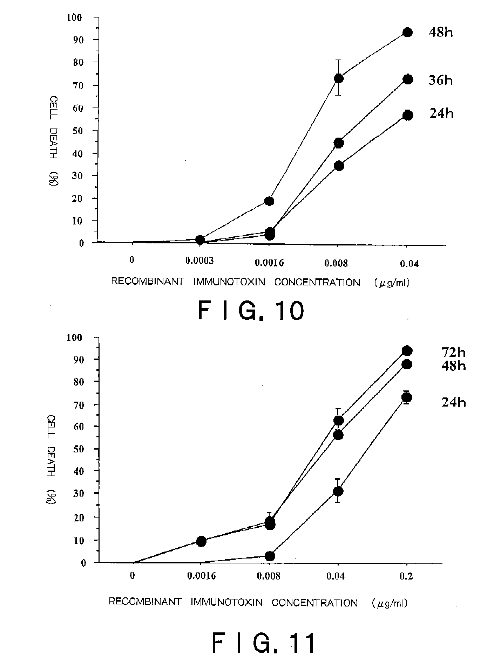

Measurements were carried out in the same manner as in Example 8 to find out whether the recombinant double chain Fv anti-FR-β PE antibody induces cytotoxicity to the FR-β expressing B300-19 cell line and the FR-β expressing HL-60 cell line. Fig. 10 demonstrates the rate of cell death (shown in the Y axis) 24 hours, 36 hours, and 48 hours after mixing the FR-β expressing B300-19 cells and the recombinant double chain Fv anti-FR-β PE chimeric antibody at various concentrations. In Fig. 10, the data are the averages of 3 experiments and the error bars demonstrate SDs.

Fig. 11 demonstrates the rate of cell death (shown in the Y axis) 24 hours, 48 hours, and 72 hours after mixing the FR-β expressing HL-60 cells and the recombinant double chain Fv anti-FR-β PE antibody at various concentrations. In Fig. 11, the data are the averages of 3 experiments and the error bars demonstrate SDs.

## Claims

1. A monoclonal antibody against folate receptor beta (FR-β).

2. The FR-β monoclonal antibody according to claim 1, which is an IgG type antibody.

3. The FR-β monoclonal antibody according to claim 1 or 2, which is produced by clone 36 cell obtained by fusion of a splenocyte from a mouse immunized with FR-β expressing B300-19 cell and a mouse myeloma cell.

4. The FR-β monoclonal antibody according to claim 1 or 2, which is produced by clone 94b cell obtained by fusion of a splenocyte from a mouse immunized with FR-β expressing B300-19 cell and a mouse myeloma cell.

5. A gene of the H chain of FR-β monoclonal antibody producing clone 36 cell consisting of the base sequence given in the following (a), (b), (c), or (d):
(a) a gene **characterized by** consisting of a base sequence shown by base number 1 to 420 in SEQ ID NO: 1 of the Sequence Listing;
(b) a gene having a base sequence which comprises partial deletions, substitutions, or additions in the base sequence shown by base number 1 to 420 in SEQ ID NO: 1 of the Sequence Listing and encoding a protein having substantially the same biological activity as a protein encoded by (a);
(c) a gene having a homology of 90% or higher with the base sequence shown by base number 1 to 420 in SEQ ID NO: 1 of the Sequence Listing and encoding a protein having substantially the same biological activity as a protein encoded by (a); or
(d) a gene which hybridizes with the base sequence shown by base number 1 to 420 in SEQ ID NO: 1 of the Sequence Listing under stringent conditions and encodes a protein having substantially the same biological activity as a protein encoded by (a).

6. A protein of the H chain of FR-β monoclonal antibody producing clone 36 cell, comprising the amino acid sequence given in the following (a), (b), or (c):
(a) a protein **characterized by** consisting of a amino acid sequence encoded by the base sequence shown by base number 1 to 420 in SEQ ID NO: 1 of the Sequence Listing;
(b) a protein having a amino acid sequence which comprises partial deletions, substitutions, or additions in the amino acid sequence encoded by the base sequence shown by base number 1 to 420 in SEQ ID NO: 1 of the Sequence Listing and having substantially the same biological activity as (a); or
(c) a protein having a homology of 90% or higher with the protein encoded by the base sequence shown by base number 1 to 420 in SEQ ID NO: 1 of the Sequence Listing and having substantially the same biological activity as (a).

7. A gene of the L chain of FR-β monoclonal antibody producing clone 36 consisting of the base sequence given in the following (a), (b), (c), or (d):
(a) a gene **characterized by** consisting of a base sequence shown by base number 1 to 381 in SEQ ID NO: 2 of the Sequence Listing;
(b) a gene having a base sequence which comprises partial deletions, substitutions, or additions in the base sequence shown by base number 1 to 381 in SEQ ID NO: 2 of the Sequence Listing and encoding a protein having substantially the same biological activity as a protein encoded by (a);
(c) a gene having a homology of 90% or higher with the base sequence shown by base number 1 to 381 in SEQ ID NO: 2 of the Sequence Listing and encoding a protein having substantially the same biological activity as a protein encoded by (a); or
(d) a gene which hybridizes with the base sequence shown by base number 1 to 381 in SEQ ID NO: 2 of the Sequence Listing under stringent conditions and encodes a protein having substantially the same biological activity as a protein encoded by (a).

8. A protein of the L chain of FR-β monoclonal antibody producing clone 36 comprising the amino acid sequence given in the following (a), (b), or (c):
(a) a protein **characterized by** consisting of the amino acid sequence encoded by the base sequence shown by base number 1 to 381 in SEQ ID NO: 2 of the Sequence Listing;
(b) a protein having a amino acid sequence which comprises partial deletions, substitutions, or additions in the amino acid sequence encoded by the base sequence shown by base number 1 to 381 in SEQ ID NO: 2 of the Sequence Listing and having substantially the same biological activity as (a); or
(c) a protein having a homology of 90% or higher with the protein encoded by the base sequence shown by base number 1 to 381 in SEQ ID NO: 2 of the Sequence Listing and having substantially the same biological activity as (a).

9. A gene of the H chain of FR-β monoclonal antibody producing clone 94b consisting the base sequence given in the following (a), (b), (c), or (d):
(a) a gene **characterized by** consisting of a base sequence shown by base number 1 to 447 in SEQ ID NO: 3 of the Sequence Listing;
(b) a gene having a base sequence which comprises partial deletions, substitutions, or additions in the base sequence shown by base number 1 to 447 in SEQ ID NO: 3 of the Sequence Listing and encoding a protein having substantially the same biological activity as a protein encoded by (a);
(c) a gene having a homology of 90% or higher with the base sequence shown by base number 1 to 447 in SEQ ID NO: 3 of the Sequence Listing and encoding a protein having substantially the same biological activity as a protein encoded by (a); or
(d) a gene which hybridizes with the base sequence shown by base number 1 to 447 in SEQ ID NO: 3 of the Sequence Listing under stringent conditions and encodes a protein having substantially the same biological activity as a protein encoded by (a).

10. A protein of the H chain of FR-β monoclonal antibody producing clone 94b consisting of the amino acid sequence given in the following (a), (b), or (c):
(a) a protein **characterized by** consisting of the amino acid sequence encoded by the base sequence shown by base number 1 to 447 in SEQ ID NO: 3 of the Sequence Listing;
(b) a protein consisting of the amino acid sequence which comprises partial deletions, substitutions, or additions in the amino acid sequence encoded by the base sequence shown by base number 1 to 447 in SEQ ID NO: 3 of the Sequence Listing and having substantially the same biological activity as (a); or
(c) a protein having a homology of 90% or higher with the protein encoded by the base sequence shown by base number 1 to 447 in SEQ ID NO: 3 of the Sequence Listing and having substantially the same biological activity as (a).

11. A gene of the L chain of FR-β monoclonal antibody producing clone 94b consisting of the base sequence given in the following (a), (b), (c), or (d):
(a) a gene **characterized by** consisting of a base sequence shown by base number 1 to 450 in SEQ ID NO: 4 of the Sequence Listing;
(b) a gene having a base sequence which comprises partial deletions, substitutions, or additions in the base sequence shown by base number 1 to 450 in SEQ ID NO: 4 of the Sequence Listing and encoding a protein having substantially the same biological activity as a protein encoded by (a);
(c) a gene having a homology of 90% or higher with the base sequence shown by base number 1 to 450 in SEQ ID NO: 4 of the Sequence Listing and encoding a protein having substantially the same biological activity as a protein encoded by (a); or
(d) a gene which hybridizes with the base sequence shown by base number 1 to 450 inSEQ ID NO: 4 of the Sequence Listing under stringent conditions and encodes a protein having substantially the same biological activity as a protein encoded by (a).

12. A protein of the L chain of FR-β monoclonal antibody producing clone 94b consisting of the amino acid sequence given in the following (a), (b), or (c):
(a) a protein **characterized by** consisting of a amino acid sequence encoded by the base sequence shown by base number 1 to 450 in SEQ ID NO: 4 of the Sequence Listing;
(b) a protein having a amino acid sequence comprises partial deletions, substitutions, or additions in the amino acid sequence encoded by the base sequence shown by base number 1 to 450 in SEQ ID NO: 4 of the Sequence Listing and having substantially the same biological activity as (a); or
(c) a protein having a homology of 90% or higher with the protein encoded by the base sequence shown by base number 1 to 450 in SEQ ID NO: 4 of the Sequence Listing and having substantially the same biological activity as (a).

13. A humanized FR-β monoclonal antibody obtained by chimerization of the gene of the H chain of clone 36 according to claim 5 with the gene of the L chain of clone 36 according to claim 7.

14. A humanized FR-β monoclonal antibody obtained by chimerization of the gene of the H chain of clone 94b according to claim 9 with the gene of the L chain of done 94b according to claim 11.

15. An FR-β antibody immunotoxin wherein the FR-β monoclonal antibody according to claim 1 or claim 2 is conjugated with a toxin.

16. The FR-β antibody immunotoxin according to claim 15, wherein said toxin is selected from the group consisting of ricin A chain, deglycosylated ricin A chain, ribosome inactivating proteins, alpha-sarcin, gelonin, aspergillin, restrictoxin, ribonuclease, epipodophyllotoxin, diphtheria toxin, and Pseudomonas exotoxin.

17. A recombinant FR-β antibody immunotoxin constructed by using the gene of the H chain of clone 36 according to claim 5 and the gene of the L chain of clone 36 according to claim 7.

18. A recombinant FR-β antibody immunotoxin constructed by using the gene of the H chain of clone 94b according to claim 9 and the gene of the L chain of clone 94b according to claim 11.

19. A conjugate of at least one biologically or chemically active molecule selected from the group consisting of enzymes, cytokines, isotopes, and chemotherapeutic agents with the FR-β monoclonal antibody according to claim 1 or claim 2.

20. A liposome containing the FR-β monoclonal antibody according to claim 1 or claim 2 and a chemotherapic agent.

21. A pharmaceutical composition comprising at least one selected from the group consisting of the FR-β antibody immunotoxin according to any one of claim 15 to claim 18, the conjugate of claim 19, and the liposome of claim 20, as active ingredient.

22. A therapeutic agent for treating a disease wherein macrophages are its major pathological condition comprising at lease one selected from the group consisting of the FR-β antibody immunotoxin according to any one of claim 15 to claim 18, the conjugate according to claim 19, and the liposome according to claim 20, as active ingredient.

23. The therapeutic agent according to claim 22, wherein said disease is a disease selected from the group consisting of rheumatoid arthritis, juvenile rheumatoid arthritis, macrophage activation syndrome, and septic shock.

24. A therapeutic agent for treating rheumatoid arthritis or juvenile rheumatoid arthritis, wherein administration form of the therapeutic agent according to claim 22 or claim 23 is a joint injection.

25. A therapeutic agent for treating leukemia comprising at least one selected from the FR-β antibody immunotoxin according to any one of claim 15 to claim 18, the conjugate according to claim 19, and the liposome according to claim 20, as active ingredient.

26. The therapeutic agent according to claim 25, wherein said leukemia is acute myeloid leukemia.


SEQUENCE LISTING

<110> Matsuyama Takami

<120> A medicine containing anti-folate receptor beta monoclonal antibody

<150> JP 05850WO

<151> 2005-4-25

<160> 15

<210> 1

<211> 420

<212> DNA

<213> Mus musculus

<221> CDS of Heavy Chain V_region of clone 36

<400> 1
   atg aaa tgg agc tgg gtc ttc ctc ttc ctc ctg tca gta act gca ggt gtc cac 54
   Met Lys Trp Ser Trp Val Phe Leu Phe Leu Leu Ser Val Thr Ala Gly Val His
   tcc cag gtt cag ctg cag cag tct gga gct gag ctg atg aag cct ggg gcc tca 108
   Ser Gln Val Gln Leu Gln Gln Ser Gly Ala Glu Leu Met Lys Pro Gly Ala Ser
   ctg aag ata tcc tgc aag gct act ggc tac aca ttc agt agc tac tgg ata gag 162
   Leu Lys Ile Ser Cys Lys Ala Thr Gly Tyr Thr Phe Ser Ser Tyr Trp Ile Glu
   tgg gta aag cag agg cct gga cat ggc ctt cag tgg att gga gaa att ttg cct 216
   Trp Val Lys Gln Arg Pro Gly His Gly Leu Gln Trp Ile Gly Glu Ile Leu Pro
   gga agt ggt agt tct aac tac aat gag aag ttc aag ggc aag gcc aca ttc act 270
   Gly Ser Gly Ser Ser Asn Tyr Asn Glu Lys Phe Lys Gly Lys Ala Thr Phe Thr
   gca gat aca tcc tcc aac aca gcc tac atg caa ctc agc agc ctg aca tct gag 324
   Ala Asp Thr Ser Ser Asn Thr Ala Tyr Met Gln Leu Ser Ser Leu Thr Ser Glu
   gac tct gcc gtc tat tac tgt gca aga cgg ggc agc tcg ggc tct tac ttt gct    378
   Asp Ser Ala Val Tyr Tyr Cys Ala Arg Arg Gly Ser Ser Gly Ser Tyr Phe Ala
   atg gac tac tgg ggt caa gga acc tca gtc acc gtc tcc tca   420
   Met Asp Tyr Trp Gly Gln Gly Thr Ser Val Thr Val Ser Ser

<210> 2
<211> 381
<212> DNA
<213> Mus musculus
<221> CDS of Light Chain V_region of clone 36
<400> 2
atg aag ttt cct tct caa ctt ctg ctc tta ctg ctg ttt gga atc cca ggc atg    54
Met Lys Phe Pro Ser Gln Leu Leu Leu Leu Leu Leu Phe Gly Ile Pro Gly Met

ata tgt gac atc cag atg aca caa tct tca tcc tcc ttt tct gta tct cta gga   108
Ile Cys Asp Ile Gln Met Thr Gln Ser Ser Ser Ser Phe Ser Val Ser Leu Gly

gac aga gtc acc att act tgc aag gca agt gag gac ata tat aat cgg tta gcc   162
Asp Arg Val Thr Ile Thr Cys Lys Ala Ser Glu Asp Ile Tyr Asn Arg Leu Ala

tgg tat cag cag aaa cca gga aat gct cct agg ctc tta ata tct ggt gca acc 216
Trp Tyr Gln Gln Lys Pro Gly Asn Ala Pro Arg Leu Leu Ile Ser Gly Ala Thr

agt ttg gaa act ggg gtt cct tta aga ttc agt ggc agt gga tct gga aag gat 270
Ser Leu Glu Thr Gly Val Pro Leu Arg Phe Ser Gly Ser Gly Ser Gly Lys Asp

tac act ctc agc att acc agt ctt cag act gaa gat att gct act tat tac tgt 324
Tyr Thr Leu Ser Ile Thr Ser Leu Gln Thr Glu Asp Ile Ala Thr Tyr Tyr Cys

caa cag tat tgg agt act ccg tgg acg ttc ggt gga ggc acc aag ctg gaa atc 378
Gln Gln Tyr Trp Ser Thr Pro Trp Thr Phe Gly Gly Gly Thr Lys Leu Glu Ile

Aaa 381
Lys

<210> 3
<211> 447
<212> DNA
<213> Mus musculus
<221> CDS of Heavy Chain V_region of clone 94b
<400> 3
atg gaa tgg acc tgg gtc att ctc ttc ctg atg gca gtg gtt aca ggg gtc aat 54
Met Glu Trp Thr Trp Val Ile Leu Phe Leu Met Ala Val Val Thr Gly Val Asn

tca gag gtt cag ctg cag cag tct ggg gca gaa ctt gtg aag cca ggg gcc tca 108

Ser Glu Val Gln Leu Gln Gln Ser Gly Ala Glu Leu Val Lys Pro Gly Ala Ser

gtc agg ttg tcc tgc aca gct tct ggc ttc aac att aaa gac acc tac atg tac   162
Val Arg Leu Ser Cys Thr Ala Ser Gly Phe Asn Ile Lys Asp Thr Tyr Met Tyr

tgg gtg aaa cag agg cct gaa cag ggc ctg gag tgg att gga agg ctt gat cct   216
Trp Val Lys Gln Arg Pro Glu Gln Gly Leu Glu Trp Ile Gly Arg Leu Asp Pro

gcg aat ggt aat act aga tat gac ccg aag ttc cag ggc aag gcc act ata aca   270
Ala Asn Gly Asn Thr Arg Tyr Asp Pro Lys Phe Gln Gly Lys Ala Thr Ile Thr

tca gac aca tcc tcc aac aca gcc tac ctg caa ctc agc agc ctg aca tct gag   324
Ser Asp Thr Ser Ser Asn Thr Ala Tyr Leu Gln Leu Ser Ser Leu Thr Ser Glu

gac act gcc gtc tat tac tgt gcg ggg gac tac ccg ata att gac tac tgg ggc   378
Asp Thr Ala Val Tyr Tyr Cys Ala Gly Asp Tyr Gly Ile Ile Asp Tyr Trp Gly

caa ggc acc act ctc aca gtc tcc tca gcc aaa acg aca ccc cca ccc gtc tat   432
Gln Gly Thr Thr Leu Thr Val Ser Ser Ala Lys Thr Thr Pro Pro Pro Val Tyr

cca ctg gcc cct gga
                    447
Pro Leu Ala Pro Gly

<210> 4

<211> 450

<212> DNA

<213> Mus musculus

<221> CDS of Light Chain V_region of clone 94b

<400> 4

atg gtt ctc atc ttg ctg ctg cta tgg gta tct ggt gag aaa ttt aaa ggt acc   54

Met Val Leu Ile Leu Leu Leu Leu Trp Val Ser Gly Glu Lys Phe Lys Gly Thr

tgt ggg gac att gtg atg tca caa tct cca tcc tcc ctg gct gtg tca gca gga   108

Cys Gly Asp Ile Val Met Ser Gln Ser Pro Ser Ser Leu Ala Val Ser Ala Gly

gag aag gtc act atg agc tgc aaa tcc agt cag agt ctg ctc aac agt aga acc   162

Glu Lys Val Thr Met Ser Cys Lys Ser Ser Gln Ser Leu Leu Asn Ser Arg Thr

cga aag acc tac ttg gct tgg tat cag cag aaa cca ggg cag tct cct aaa ctg   216

Arg Lys Thr Tyr Leu Ala Trp Tyr Gln Gln Lys Pro Gly Gln Ser Pro Lys Leu

ctg atc tac tgg gca tcc act agg gaa tct ggg gtc cct gat cgc ttc aca ggc   270

Leu Ile Tyr Trp Ala Ser Thr Arg Glu Ser Gly Val Pro Asp Arg Phe Thr Gly

agt gga tct ggg aca gag ttc act ctc acc atc agc agt gtg cag gct gaa gac   324

Ser Gly Ser Gly Thr Glu Phe Thr Leu Thr Ile Ser Ser Val Gln Ala Glu Asp

ctg gca gtt tat tac tgc aag caa tct tat aat ctg tgg acg ttc ggt gga ggc   378

Leu Ala Val Tyr Tyr Cys Lys Gln Ser Tyr Asn Leu Trp Thr Phe Gly Gly Gly

acc aag ctg gaa atc aaa cgg gct gat gct gca cca act gta tcc atc ttc ccg   432
Thr Lys Leu Glu Ile Lys Arg Ala Asp Ala Ala Pro Thr Val Ser Ile Phe Pro

cca tcc agt aag ctg gga
                        450
Pro Ser Ser Lys Leu Gly

<210> 5
<211> 26
<212> DNA
<213> Artificial Sequence
<220>
<223> Primer for PCR
<400> 5
agaaagacat ggtctggaaa tggatg   26

<210> 6
<211> 28
<212> DNA
<213> Artificial Sequence
<220>
<223> Primer for PCR
<400> 6
gactgaactc agccaaggag ccagagtt   28

<210> 7

<211> 35

<212> DNA

<213> Artificial Sequence

<220>

<223> Primer for PCR

<400> 7

cagaggcctg aacagtgtct ggagtggatt ggaag   35

<210> 8

<211> 35

<212> DNA

<213> Artificial Sequence

<220>

<223> Primer for PCR

<400> 8

cttccaatcc actccagaca ctgttcaggc ctctg   35

<210> 9

<211> 17

<212> DNA

<213> Artificial Sequence

<220>

<223> M13 reverse primer

<400> 9

caggaaacag ctatgac   17

<210> 10

<211> 41

<212> DNA

<213> Artificial Sequence

<220>

<223> Annealing primer

<400> 10

taagaaggag atatacatat ggaggttcag ctgcagcagt c   41

<210> 11

<211> 44

<212> DNA

<213> Artificial Sequence

<220>

<223> Annealing primer

<400> 11

gccctcggga cctccggaag cttttgagga gactgtgaga gtgg   44

<210> 12

<211> 20

<212> DNA

<213> Artificial Sequence

<220>

<223> T7 promoter primer

<400> 12

taatacgact cactataggg   20

<210> 13

<211> 41

<212> DNA

<213> Artificial Sequence

<220>

<223> Annealing primer

<400> 13

taagaaggag atatacatat ggacattgtg atgtcacaat c   41

<210> 14

<211> 57

<212> DNA

<213> Artificial Sequence

<220>

<223> Annealing primer

<400> 14

gctttgttag cagccgaatt cctatttgat ttccagcttg gtgccacaac cgaacgt   57

<210> 15

<211> 20

<212> DNA

<213> Artificial Sequence

<220>

<223> T7 promoter primer

<400> 15

taatacgact cactataggg   20